# EUROPEAN PATENT SPECIFICATION

(11) **EP 2 783 625 B1**
(45) Date of publication and mention of the grant of the patent: **30.09.2015**
(21) Application number: 14156982.2
(22) Date of filing: 27.02.2014
(51) Int. Cl.: A61B 5/00, H04W 4/02

(54) **Information processing apparatus and warning issuing method**
Informationsverarbeitungsvorrichtung und Warnungsausgabeverfahren
Appareil de traitement d'informations et procédé d'émission d'avertissement

(30) Priority: 28.03.2013 JP 2013069001
(43) Date of publication of application: 01.10.2014
(73) Proprietor: FUJITSU LIMITED, Kawasaki-shi Kanagawa 211-8588 (JP)
(72) Inventor: KASAMA, Kouichirou, Nakahara-ku, Kanagawa 211-8588 (JP)
(74) Representative: Wilding, Frances Ward

(56) References cited:
- EP-A1- 1 916 850
- DE-A1- 4 209 336
- JP-A- 2002 293 240
- JP-A- 2008 140 227

## Description

### FIELD

The embodiments discussed herein are related to an information processing apparatus, a warning issuing method, and a storage medium.

### BACKGROUND

For example, a sleep state determination device having a brain activity measurement unit and a sleep state determination unit so as to determine a sleep state of a subject is known. This sleep state determination device measures indexes reflecting brain activity that indicates of levels of changes in brain activity states of a sleeping subject for arbitrary units of time. The sleep state determination unit determines a sleep state at an arbitrary time included in a time frame on the basis of the brain activity measurement unit and an index reflecting the brain activity measured by the brain activity measurement unit for units of time in the time frame that includes one cycle of sleep. This configuration aims to raise an alarm during REM (Rapid Eye Movement) sleep, in which people can wake up with a comfortable feeling, by determining a sleep state with high accuracy (See Patent Document 1 for example).

A mobile terminal that controls alarm raising states appropriately is also known. This mobile terminal includes an acceleration sensor, an alarm unit, a time determination unit, and a control unit. The acceleration sensor detects whether or not the mobile terminal is moving. The alarm unit raises an alarm at a set time. The time determination unit determines a first time on the basis of information obtained by the acceleration sensor, determines a second time on the basis of the time at which the alarm unit is operated, and performs determination by comparing the first and second times. The control unit performs controls so as to change operations of the alarm unit in accordance with results of determination by the time determination unit (see Patent Document 2 for example) .

Also, a train-connection guidance system is known that detects delays in a real time manner so as to raise an alarm at a more accurate arrival time. In this train-connection guidance system, a mobile telephone with a Global Positioning System (GPS) measures a current position periodically. The mobile telephone compares the position information of the obtained current position and time table information that is provided by a train-connection guidance site with position information of each station on the train line so as to correct an arrival time by detecting a delay time in a real time manner even when a train delays (see Patent Document 3 for example).

A method has also been proposed in which a change index or the like obtained by frequency analysis of pulse wave intervals is used for estimating sleep state changes of a sleeping subject in a real time manner (see non Patent Document 1 for example). A method is also known in which whether or not a user is on a conveyance such as a train or the like is detected by an acceleration sensor provided to a mobile terminal device (see non Patent Document 2 for example).
Patent Document 1: Japanese Laid-open Patent Publication No. 2006-192152
Patent Document 2: Japanese Laid-open Patent Publication No. 2011-109432
Patent Document 3: Japanese Laid-open Patent Publication No. 2010-231303
Non Patent Document 1: "Sleep state estimation method using pulse wave and its application" by Akihisa Moriya, et al. Human Interface Society, Vol. 10, No.2, 2008, pp. 77-84
Non Patent Document 2: "Implementation and evaluation of transfer guidance application based on human context recognizer" by Yuzo Okamoto, et al. the 9th Forum on Information Technology, volume 4, pp. 67-72

### SUMMARY

According to an aspect, it is an object of the present invention to reduce cases where warnings are not noticed when an information processing apparatus indicates that a destination has gotten closer, by avoiding cases where the amount of power is insufficient due to sleep depth measurement.

According to an aspect of the embodiments, an information processing apparatus includes a heartbeat sensor configured to measure heartbeat of a user, an acceleration sensor configured to detect acceleration, a position information detection device configured to detect position information, and a warning issuing device configured to issue a warning. The information processing apparatus further includes a storage device configured to hold a first cycle based on transition times of sleep depths for one cycle, and a processor. The processor is configured to detect boarding of the user on a conveyance on the basis of the acceleration, and to activate the heartbeat sensor when the boarding has been detected. The processor is configured to detect a transition to sleep of the user and a transition time to the sleep on the basis of heartbeat measured by the heartbeat sensor, and to stop driving of the heartbeat sensor when the transition has been detected. The processor is configured to make the warning issuing device issue a warning when it has been determined that a distance between a position represented by the position information detected by the position information detection device and a destination is shorter than a prescribed value and that a sleep depth of the user is shallow on the basis of the first cycle and a transition time to the sleep.

### BRIEF DESCRIPTION OF DRAWINGS

FIG. 1 illustrates an example of a configuration of a mobile terminal according to a first embodiment;
FIG. 2 is a block diagram showing functions of the mobile terminal according to the first embodiment;
FIG. 3 illustrates an example of a display example according to the first embodiment;
FIG. 4 illustrates a concept of determination performed by an alarm raising determination unit according to the first embodiment;
FIG. 5 illustrates an example of an alarm table for determination performed by the alarm raising determination unit according to the first embodiment;
FIG. 6 illustrates an example of a sleep depth time management table according to the first embodiment;
FIG. 7 illustrates an example of a sleep depth time estimation table according to the first embodiment;
FIG. 8 is a flowchart explaining a warning issuing process according to the first embodiment;
FIG. 9 is a flowchart explaining a warning issuing process according to the first embodiment;
FIG. 10 is a flowchart explaining a distance determination process according to the first embodiment;
FIG. 11 is a flowchart explaining an alarm determination process according to the first embodiment;
FIG. 12 illustrates an example of a sleep depth time estimation table according to a second embodiment;
FIG. 13 is a flowchart explaining a warning issuing process according to the second embodiment;
FIG. 14 is a flowchart explaining a warning issuing process according to the second embodiment;
FIG. 15 is a flowchart explaining an alarm determination process according to the second embodiment;
FIG. 16 illustrates an example of a sleep depth time estimation table according to a third embodiment;
FIG. 17 is a flowchart explaining a warning issuing process according to the third embodiment; and
FIG. 18 is a flowchart explaining a warning issuing process according to the third embodiment.

### DESCRIPTION OF EMBODIMENTS

The above described conventional information processing apparatuses have the following problems. According to the method that determines sleep states with high accuracy so as to raise an alarm during REM sleep, which brings a comfortable waking up feeling, it is assumed that alarms are noticed because sleep depths are shallow. However, this method requires that a heartbeat sensor be kept in operation in order to monitor sleep depths continuously, sometimes resulting in insufficient power with respect to the power consumption in mobile terminals. According to the method in which a user sets a time to raise an alarm, the method is not convenient in cases of returning home, where a time at which the user arrives at the station nearest to his or her house is not consistent, or in cases of business journeys. Even when train delay is obtained in a real time manner, alarms are sometimes not noticed depending upon sleep depths.

Preferred embodiments of the present invention will be explained with reference to accompanying drawings.

### (First embodiment)

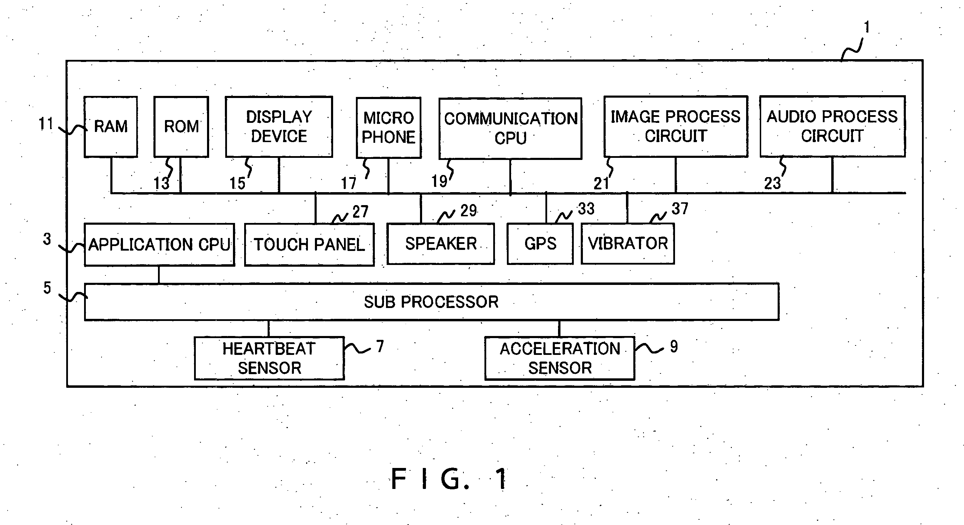

Hereinafter, by referring to the drawings, explanations will be given for a warning issuing method in a mobile terminal 1 according to a first embodiment. FIG. 1 illustrates an example of a configuration of the mobile terminal 1. As illustrated in FIG. 1, the mobile terminal 1 includes an application central processing unit (CPU) 3, a sub processor 5, a heartbeat sensor 7, and an acceleration sensor 9. The mobile terminal 1 include a random access memory (RAM) 11, a read only memory (ROM) 13, a display device 15, a microphone 17, and a communication CPU 19. Also, the mobile terminal 1 includes an image process circuit 21, an audio process circuit 23, a touch panel 27, a speaker 29, a GPS 33, and a vibrator 37. The above constituents in the mobile terminal 1 are connected through, for example, a system bus and information can be exchanged between them.

The mobile terminal 1 is, for example, an information processing apparatus such as a multifunctional mobile phone, an mobile information terminal, etc. The application CPU 3 is a processor that performs processes in accordance with an application program executed by the mobile terminal 1. The sub processor 5 is a processor that is kept continuously in an operating state even during, for example, the standby mode of the mobile terminal 1. The heartbeat sensor 7 is a detection device such as, for example, a millimeter wave sensor etc. and can detect heartbeat in a contactless manner. The acceleration sensor 9 is a detection device that measures, for example, 3-dimensional acceleration.

The RAM 11 is a storage device that can write and read information on an as-needed basis, and for example is used for storing a program for controlling the operations of the mobile terminal 1 and used as a working area when necessary for executing a program. The ROM 13 is a readable storage device, and stores beforehand, for example, a program for controlling operations of the mobile terminal 1. The display device 15 is a device that displays information such as, for example, a liquid crystal display device or the like. The microphone 17 is a device that collects audio.

The communication CPU is a processor that controls, for example, telephone calls or communications via the GPS 33. The image process circuit 21 is a circuit that performs an image process related to an image or the like obtained by a camera 25. The camera 25 is an image pickup device. The audio processing circuit 23 is a circuit that performs a signal processing related to audio that is output from the speaker 29 and audio that is collected by the microphone 17. The speaker 29 is an output device that outputs audio.

The touch panel 27 is attached to for example the display device 15 and detects contact at a position in accordance with displayed information so as to input information. The GPS 33 is a device that obtains a current position by using the distances from satellites. The vibrator 37 is a device that oscillates vibrations. The mobile terminal 1 may be provided with for example a camera that picks up an image and a communication device that perform short-distance wireless communications in accordance with various protocols such as Bluetooth (trademark), WiFi (trademark), or the like in accordance with the above constituents.

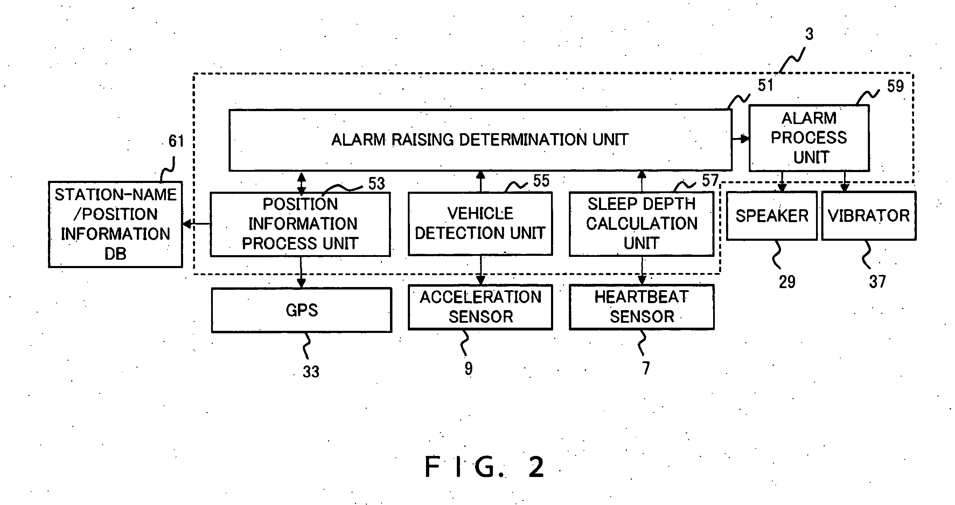

FIG. 2 is a block diagram showing functions of the mobile terminal 1. As illustrated in FIG. 2, the application CPU 3 reads and executes an application program stored in, for example, the RAM 11 so as to implement the illustrated functions. Specifically, the application CPU 3 is provided with an alarm raising determination unit 51, a position information processing unit 53, a conveyance detection unit 55, a sleep depth calculation unit 57, and an alarm processing unit 59. Also, the RAM 11 stores, for example, station-name/position information database (DB) 61, thresholds necessary for processes that will be describe later, and the like.

The alarm raising determination unit 51 determines whether or not to raise an alarm in accordance with a condition obtained by the position information processing unit 53, the conveyance detection unit 55, and the sleep depth calculation unit 57. More specifically, when the conveyance detection unit 55 has detected boarding, it requests, from the position information processing unit 53, the position information of a current station, obtains the time, and determines whether or not to raise an alarm in accordance with an alarm table 80 and a sleep depth time management table 90, which will be described later. An alarm is a warning that is issued by the speaker 29, the vibrator 37, or the like. Also, the alarm raising determination unit 51 requests that the alarm processing unit 59 raise an alarm and requests that the alarm processing unit 59 stop the alarm when the alarm raising determination unit 51 has determined to raise an alarm.

The position information processing unit 53 refers to the current position obtained by the GPS 33 and the station-name/position information DB 61 so as to determine a route which is considered that the user is boarding and calculates the distance to the destination. More specifically, when a destination has been obtained, the position information processing unit 53 refers to the station-name/position information DB 61 so as to obtain the position information of the destination, calculates the distance between the current position and the destination, compares the calculated distance and threshold in the alarm table 80, and reports the result to the alarm raising determination unit 51.

The conveyance detection unit 55 detects whether or not boarding occurred in the basis of the acceleration detected by the acceleration sensor 9. More specifically, the conveyance detection unit 55 samples measurement values detected by the acceleration sensor 9 so as to determine whether boarding has occurred, and reports the determination result to the alarm raising determination unit 51. When the conveyance detection unit 55 has detected that the boarding has occurred, it may make a request to activate a display example 65. Whether or not the boarding has occurred can be detected by using various known techniques including, for example, the method described in non-Patent Document 2.

The sleep depth calculation unit 57 determines a sleep state on the basis of a detection result of the heartbeat sensor 7 and calculates a sleep depth. More specifically, the sleep depth calculation unit 57 samples the value of the heartbeat sensor 7 so as to determine a sleep depth. The sleep depth calculation unit 57 stores, in the sleep depth time management table 90, which will be described later, transition times to the respective sleep depths from REM sleep->shallow non-REM sleep->deep non-REM sleep->shallow non-REM sleep->REM sleep. When boarding has been detected by the conveyance detection unit 55, the sleep depth calculation unit 57 activates the heartbeat sensor 7 so as to stop the heartbeat sensor at a prescribed timing such as a time when REM sleep is detected. Further, the sleep depth calculation unit 57 generates a sleep depth time estimation table 100, which will be explained later, on the basis of the REM sleep starting time detected after the detection of boarding.

The set from REM sleep->shallow non-REM sleep->deep non-REM sleep->shallow non-REM sleep is referred to as one cycle of sleep hereinafter. Measurement of a sleep cycle means calculation of a transition cycle from transition times by detecting transitions of sleep depths of REM sleep, shallow non-REM sleep, deep non-REM sleep, etc. included in one sleep cycle. Sleep depths and transition to sleep can be detected by known methods such as for example the method described in a non-Patent Document 1.

The alarm processing unit 59 raises an alarm through the speaker 29 or the vibrator 37 and performs a process of stopping the alarm that has been raised, in accordance with the determination by the alarm raising determination unit 51. The station-name/position information DB 61 is information in which station names and position information (such as latitude and longitude) thereof are stored in an associated manner. Position information desirably includes latitude and longitude, the distance from the terminal station, and the like. Station names are desirably stored in a state that they are associated with, for example, routes.

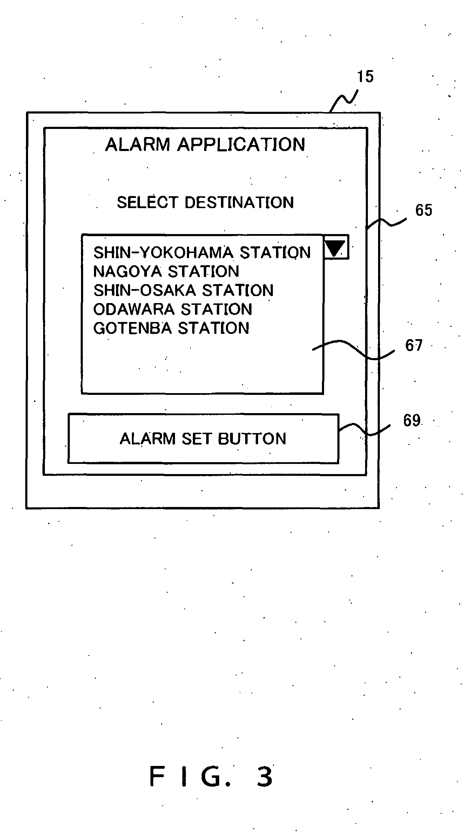

FIG. 3 illustrates an example of the display example 65 displayed in the display device 15. The display example 65 is an example of a window displayed when, for example, an application program for issuing a warning is activated according to the present embodiment. The display example 65 may be displayed when a sleep cycle is measured during sleep and when boarding is detected by the conveyance detection unit 55 is detected.

It is also possible to display, in station name 67, station names of a route that is used highly frequently when a sleep cycle is measured. When boarding has been detected, the position information processing unit 53 determines, as illustrated in the display example 65, the route on the basis of the station-name/position information DB 61 and position information detected by the GPS 33. As illustrated in station name 67, the position information processing unit 53 may search the station-name/position information DB 61 for the station name of the route so as to display it. An instruction input unit 69 is an input unit for selecting and contacting a station name in the station name 67 so as to set a destination.

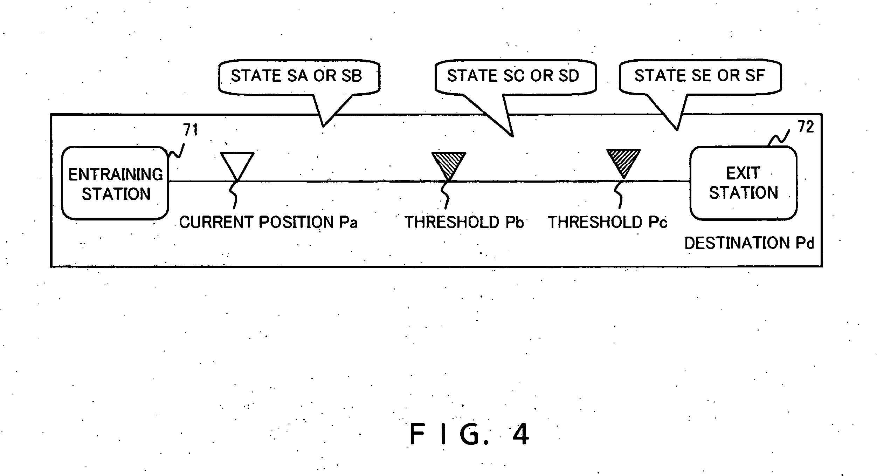

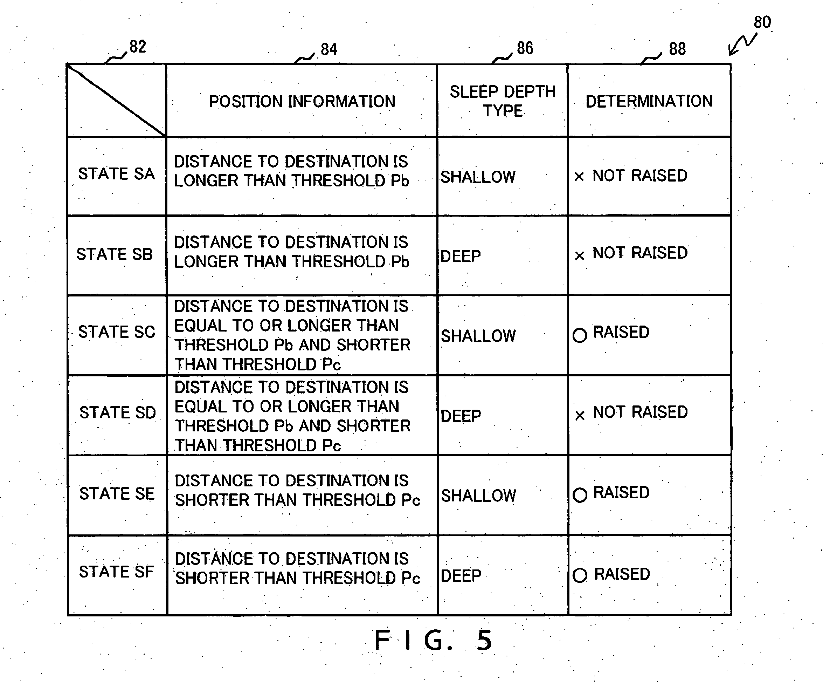

FIG. 4 schematically illustrates determination performed by the alarm raising determination unit 51. FIG. 5 illustrates an example of the alarm table 80 for determination by the alarm raising determination unit 51. As illustrated in FIG. 4, an entraining station 71 is a station that has been determined to be a station at which a user boarded, and an exit station 72 represent a station that was input as a destination. The distance between the entraining station 71 and the exit station 72 is described as distance Pd. Thresholds Pb and Pc (Pc<Pb<Pd) represent prescribed distances from the exit station 72. Current position Pa is a current position of the mobile terminal 1. States SA and SB represent states of an alarm that can be set when the distance between a position at which a sleep state is detected and the exit station 72 is equal to or longer than threshold Pb. States SC and SD represent states of an alarm that can be set when the distance to the exit station 72 is shorter than threshold Pb and equal to or longer than threshold Pc. States SE and SF represent states of an alarm that can be set when the distance to the exit station 72 is shorter than threshold Pc.

As illustrated in FIG. 5, the alarm table 80 includes a state type 82, position information 84, a sleep depth type 86, and determination 88, and is stored in for example the RAM 11. The state type 82 represents one of the above states SA through SF. The position information 84 is a distance to the exit station 72 that corresponds to the state type 82. The sleep depth type 86 is a type representing whether the sleep depth is shallow. The determination 88 is information representing whether or not an alarm is raised.

In the alarm table 80 according to the present embodiment, it is set as the determination 88 to "raise" an alarm (states SE and SF) regardless of the sleep depth type 86 when the distance to the exit station 72 is shorter than threshold Pc. It is set to "raise" an alarm when the sleep depth type 86 is "shallow" in a case when the distance to the exit station 72 is shorter than threshold Pb. It is set that an alarm is "not raised" when the distance is equal to or longer than threshold Pb (states SA and SB) and when the distance is shorter than threshold Pb and the sleep depth type 86 is "deep" (state SD). It is also possible to assume that the sleep depth type 86 that is "shallow" means a case where the sleep is determined to be REM sleep and the sleep depth type 86 that is "deep" is a case where the sleep is determined to be shallow non-REM sleep or deep non-REM sleep.

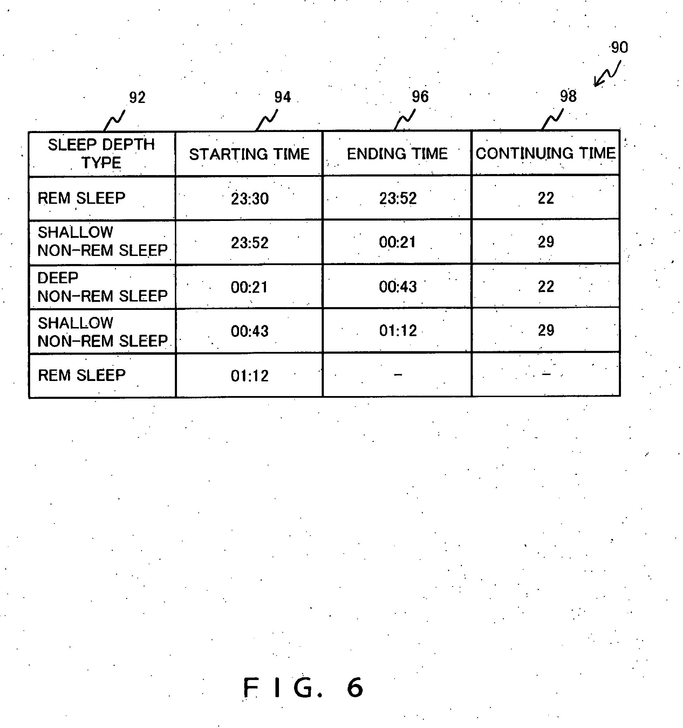

FIG. 6 illustrates an example of the sleep depth time management table 90. The sleep depth time management table 90 is for example collection of pieces of information obtained by determining sleep from the night of the day before a day in which a warning is to be issued according to the present embodiment, and is stored in for example RAM 11. The sleep depth time management table 90 is data that functions as a reference when the alarm raising determination unit 51 determines sleep. As illustrated in FIG. 6, the sleep depth time management table 90 includes a sleep depth type 92, a starting time 94, an ending time 96, and a continuing time 98. The sleep depth type 92 is a type representing whether or not sleep is deep. The starting time 94 is a time at which sleep corresponding to the sleep depth type 92 is determined to have started. The ending time 96 is a time at which sleep corresponding to the sleep depth type 92 is determined to have ended. The continuing time 98 is a period of time between the starting time 94 and the ending time 96. In the two successive sleep depth types 92, the ending time 96 and the starting time 94 coincide.

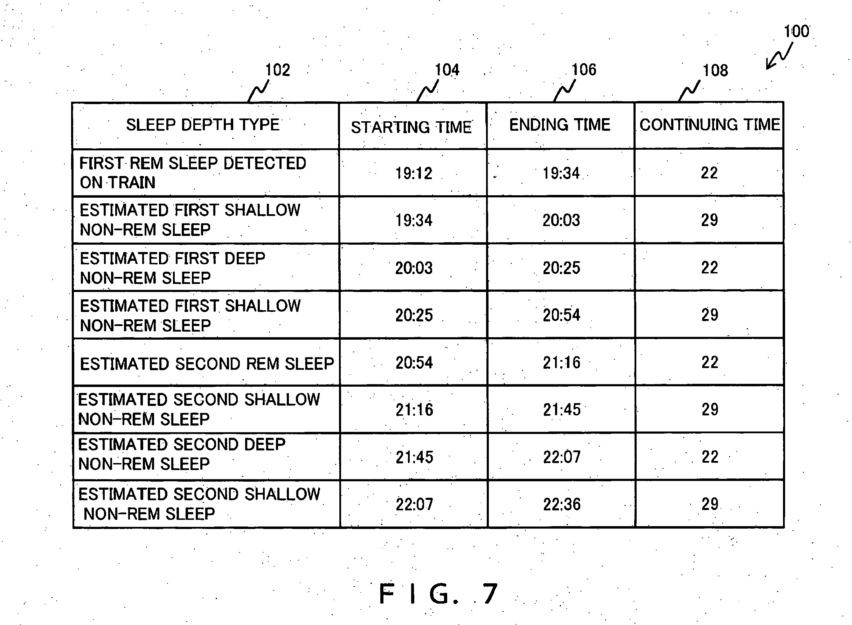

FIG. 7 illustrates an example of the sleep depth time estimation table 100. The sleep depth time estimation table 100 is information representing prediction of transitions of sleep depths after the detection of a sleep state after the boarding was detected, and is generated on the basis of the sleep depth time management table 90 and the detection by the sleep depth calculation unit 57. The sleep depth time estimation table 100 is stored in for example the RAM 11. In the present embodiment, the sleep depth time estimation table 100 is information referred to when the alarm raising determination unit 51 determines whether or not to raise an alarm.

As illustrated in FIG. 7, the sleep depth time estimation table 100 includes a sleep depth type 102, a starting time 104, an ending time 106, and a continuing time 108. The sleep depth type 102 is a type representing whether or not a sleep depth is shallow. The starting time 104 is time at which REM sleep in the first cycle after boarding is determined to have started or a time at which sleep corresponding to the sleep depth type 102 is predicted to start. The ending time 106 is a time at which sleep corresponding to the sleep depth type 102 is predicted to end. The continuing time 108 is a period of time between the starting time 104 and the ending time 106.

In the sleep depth time estimation table 100, when the sleep depth type 102 is "first-cycle REM sleep detected on a train", the starting time 104 is a time at which REM sleep was determined to have started actually after the boarding was detected by the conveyance detection unit 55. The ending time 106 after that and the starting time 104 and the ending time 106 of different sleep depth type 102 are predicted times that are calculated by sequentially adding the corresponding continuing time 98 measured by the sleep depth time management table 90. Specifically, the sleep depth time estimation table 100 is generated on an assumption that the sleep depth type 102 transitions at timing similar to that of the sleep depth time management table 90 after detection of a sleep state after boarding.

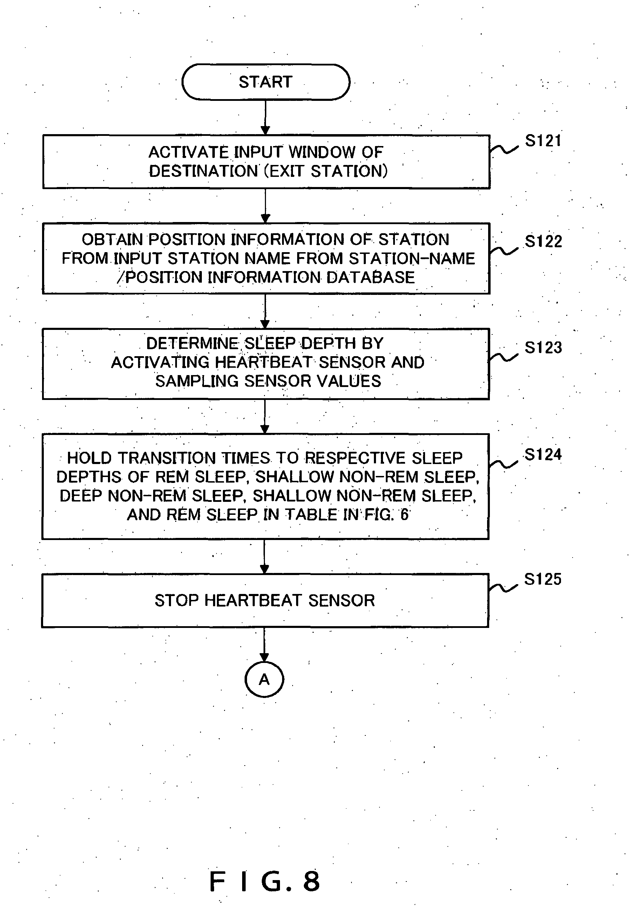

Hereinafter, by referring to FIG. 8 through FIG. 11, operations of the mobile terminal 1 according to the present embodiment will further be explained. The processes below performed by the mobile terminal 1 are implemented by the application CPU 3 by reading a prescribed program from for example the RAM 11 and executing the program. However, explanations will be given on an assumption that the processes are executed by the respective functions explained with reference to FIG. 2.

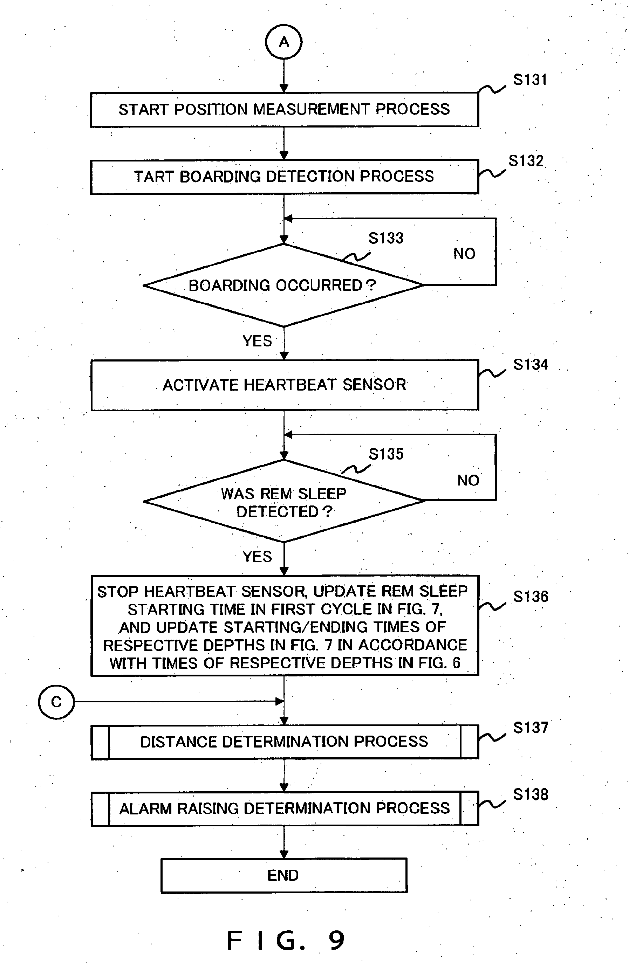

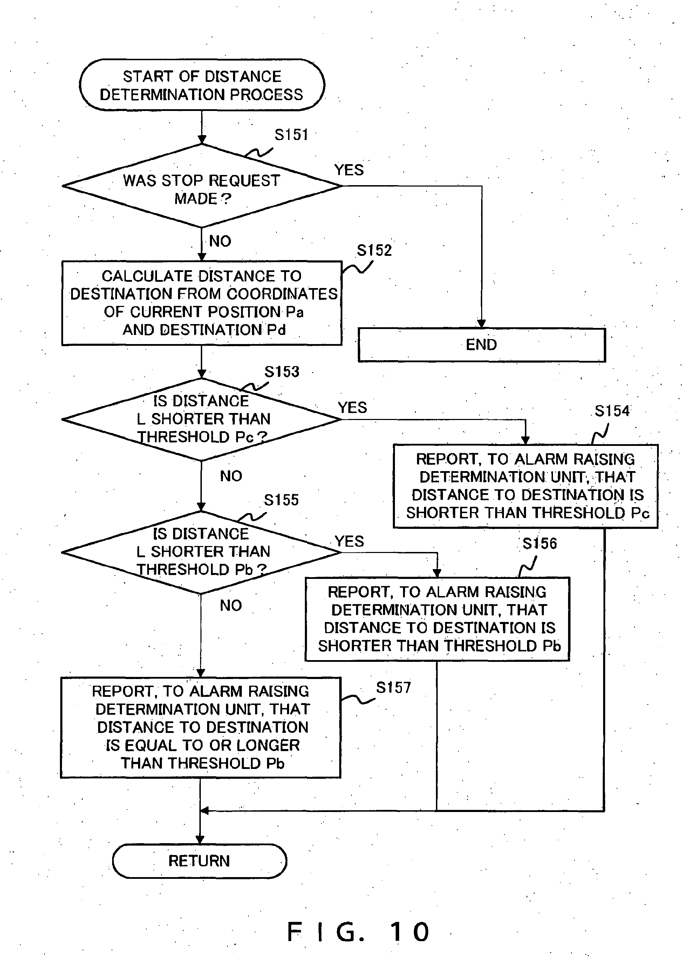

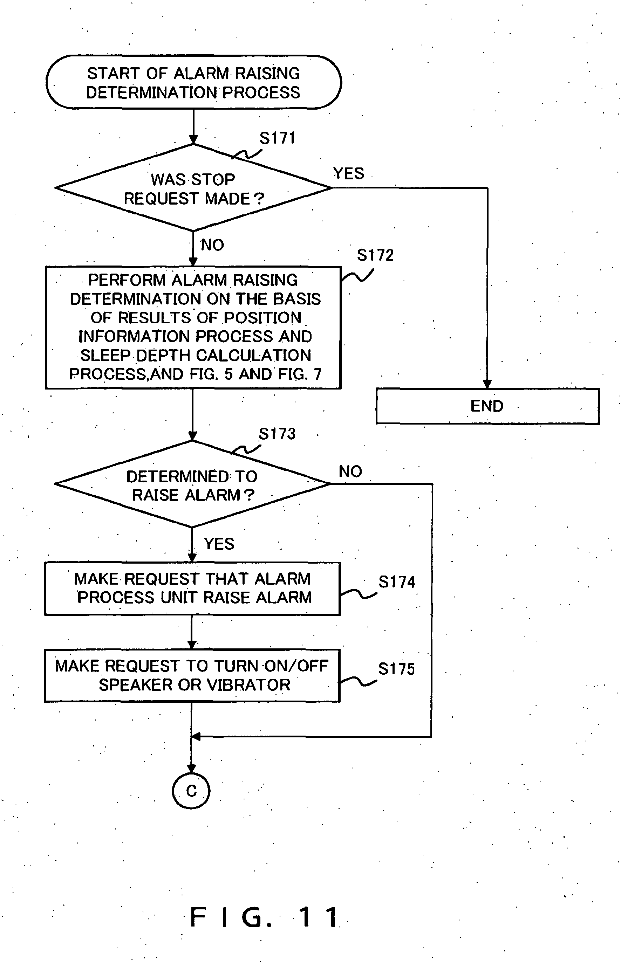

FIG. 8 and FIG. 9 are flowcharts showing a warning issuing process performed by the mobile terminal 1 according to the present embodiment. FIG. 10 is a flowchart showing a distance determination process. FIG. 11 is a flowchart showing an alarm determination process.

As illustrated in FIG. 8, when an application program for issuing a warning according to the present embodiment is activated by for example an input through the touch panel 27, the position information processing unit 53 makes the display device 15 display the display example 65 (S121). The position information processing unit 53 obtains, in accordance with the station-name/position information DB 61, the position information of a station input by the instruction input unit 69 from the station name 67 (S122). The sleep depth calculation unit 57 activates the heartbeat sensor 7 and determines the sleep depth by sampling sensor values that can be obtained (S123). The determination of a sleep depth can be performed by using a known method such as for example a method described in non-Patent Document 1.

The sleep depth calculation unit 57 holds transition times to the respective sleep depths of REM sleep->shallow non-REM sleep->deep non-REM sleep->shallow non-REM sleep->REM sleep in the sleep depth time management table 90 in FIG. 6 (S124). When the sleep depth calculation unit 57 has obtained the transition times of sleep depths for one cycle, it stops the heartbeat sensor 7 (S125). The sleep depth calculation unit 57 may continue obtaining transition times to sleep depths until awakening so as to stop the heartbeat sensor 7 when awakening is detected. For this, the sleep depth time management table 90 may hold times obtained in the latest one cycle or may calculate and hold the average or the like.

As illustrated in FIG. 9, the 53 starts a measurement process of the current position (S131). Specifically, the position information processing unit 53 uses the GPS 33 to perform measurement processes for example once for a prescribed period of time, and obtains current positions. The conveyance detection unit 55 starts detection of whether or not boarding on a conveyance such as for example a train or the like occurred (S132). The conveyance detection unit 55 samples acceleration obtained by the acceleration sensor 9 so as to repeat the determination until boarding is detected (NO in S133), and detects whether or not boarding occurred.

When the conveyance detection unit 55 has detected boarding (YES in S133), the sleep depth calculation unit 57 activates the heartbeat sensor 7 (S134). At this moment, the position information processing unit 53 may display the display example 65 for inputting a destination. The sleep depth calculation unit 57 repeats determination until REM sleep is detected on the basis of values of the heartbeat of the user detected by the heartbeat sensor 7 (NO in S135). When the sleep depth calculation unit 57 has detected REM sleep (YES in S135), it stops the heartbeat sensor 7, and updates the time at which it detected the starting time 104 of the REM sleep in the first cycle in the sleep depth time estimation table 100 in FIG. 7. Also, the sleep depth calculation unit 57 sets prediction of transition times to sleep depths in the sleep depth time estimation table 100 on the basis of the detected starting time and the sleep depth time management table 90 illustrated in FIG. 6 (S136).

Next, the position information processing unit 53 performs a distance determination process (S137). Also, the alarm raising determination unit 51 performs an alarm raising determination process (S138). The distance determination process and the alarm raising determination process will be described in detail below.

As illustrated in FIG. 10, the alarm raising determination unit 51 determines whether or not a request to stop an application program was made (S151). At this moment, the alarm raising determination unit 51 may display information for making a request to stop. When a request to stop was made, the alarm raising determination unit 51 stops the speaker 29, the vibrator 37, or the like when an alarm has been raised, and terminates the process (YES in S151). When a request to stop was not made (NO in S151), the position information processing unit 53 first calculates distance L to the destination on the basis of the coordinates (latitude and longitude) of current position Pa the destination (exit station 72) (S152).

The position information processing unit 53 determines whether or not distance L is shorter than threshold Pc (S153). When it is shorter (YES in S153), the position information processing unit 53 reports to the alarm raising determination unit 51 that distance L is shorter than threshold Pc (S154), and the process returns to S151. When it is not shorter (NO in S153), the position information processing unit 53 determines whether or not distance L is shorter than threshold Pb (S155). When it is shorter (YES in S155), the position information processing unit 53 reports to the alarm raising determination unit 51 that distance L is shorter than threshold Pb (S156), and the process returns to s151. When it is not shorter (NO in S155), the position information processing unit 53 reports to the alarm raising determination unit 51 that distance L is greater than threshold Pb (S157), and the process returns to S151.

As illustrated in FIG. 11, the alarm raising determination unit 51 determines whether or not a stopping request of an application program was made (S171). When a stopping request was made and when an alarm has been raised, the alarm raising determination unit 51 stops the alarm, and the process is terminated (YES in S171). When a stopping request was not made (NO in S171), the alarm raising determination is performed (S172) in accordance with the determination process results by the position information processing unit 53 and the sleep depth calculation unit 57, the alarm table 80 illustrated in FIG. 5, and the sleep depth time estimation table 100 illustrated in FIG. 7. For example, when a distance measured by the position information processing unit 53 and the sleep depths measured by the sleep depth time estimation table 100 are one of states SC, SE, and SF of the alarm table 80, the alarm raising determination unit 51 determines to make the alarm processing unit 59 raise an alarm. In other cases, it determines not to raise an alarm.

In cases where the determination is not determination to raise an alarm, the alarm raising determination unit 51 makes the process return to S171 (NO in S173). In cases where the determination is determination to raise an alarm (YES in S173), the alarm raising determination unit 51 issues an alarm raising request to the alarm processing unit 59 (S174). The alarm processing unit 59 issues an ON-OFF request to the speaker 29 or the vibrator 37 (S175), and makes the process return to S137 in FIG. 8 (S175).

As described in detail above, according to the mobile terminal 1 of the first embodiment, the sleep depth calculation unit 57 first measures timings of transitions to sleep depths in at least one cycle, stops the heartbeat sensor 7, and generates the sleep depth time management table 90. This measurement may be performed during the latest sleep of a user carrying the mobile terminal 1 before he or she boards a conveyance.

When the conveyance detection unit 55 has detected boarding, the sleep depth calculation unit 57 drives the heartbeat sensor 7. When the sleep depth calculation unit 57 has detected a start of REM sleep in the first cycle, it updates the sleep depth time estimation table 100 on the basis of the starting time and the sleep depth time management table 90. Also, the sleep depth calculation unit 57 stops the heartbeat sensor 7. The position information processing unit 53 obtains the current position by using the GPS 33 for example once in a prescribed period of time, and calculates the distance to the destination that has been obtained beforehand.

The alarm raising determination unit 51 determines whether or not the calculated distance and a sleep depth estimated by the sleep depth time estimation table 100 meet a prescribed condition in the alarm table 80. When it does, the alarm raising determination unit 51 issues a warning by using the alarm processing unit 59. Specifically, the alarm raising determination unit 51 issues a warning regardless of a sleep depth when the distance from the destination is shorter than threshold Pc. Also, when the distance from the destination is shorter than Pb, which is greater than threshold Pc, and when it has been determined that a sleep depth is shallow, a warning is issued.

As described above, in the warning issuing method by the mobile terminal 1 according to the present embodiment, a warning is issued when a distance to a destination and a sleep depth meet a prescribed condition, making it possible to reduce cases where users do not notice warnings. By combining pieces of information obtained from the application CPU 3, the heartbeat sensor 7 and the acceleration sensor 9 as described above, a situation favorable for issuing a waking warning can be determined. This makes it possible for the mobile terminal 1 to monitor the distance between a user's home and the nearest station and a sleep state so as to output an alarm when a sleep has been determined to be REM sleep after the user has gotten close to the nearest station. This makes it possible to raise an alarm during REM sleep when the user is close to his or her home even when the arrival time is not known.

The mobile terminal 1 issues a warning when sleep has been determined to be shallow, making it possible to wake up users in a good mood. It is also possible to beforehand measure transitions to sleep depths in at least one cycle during sleep in, for example, a home or the like and predict transitions to sleep depths on the basis of timings of transitioning to sleep depths of the user. Further, it is possible to predict transitions to sleep depths highly accurately because predicted times are updated by detecting the REM sleep in the first cycle after boarding. Also, the heartbeat sensor 7 is not driven before the detection of boarding and after the detection of the REM sleep in the first cycle. Accordingly, it is not necessary to keep the heartbeat sensor 7 driven continuously, making it possible to suppress power consumption.

### (Second Embodiment)

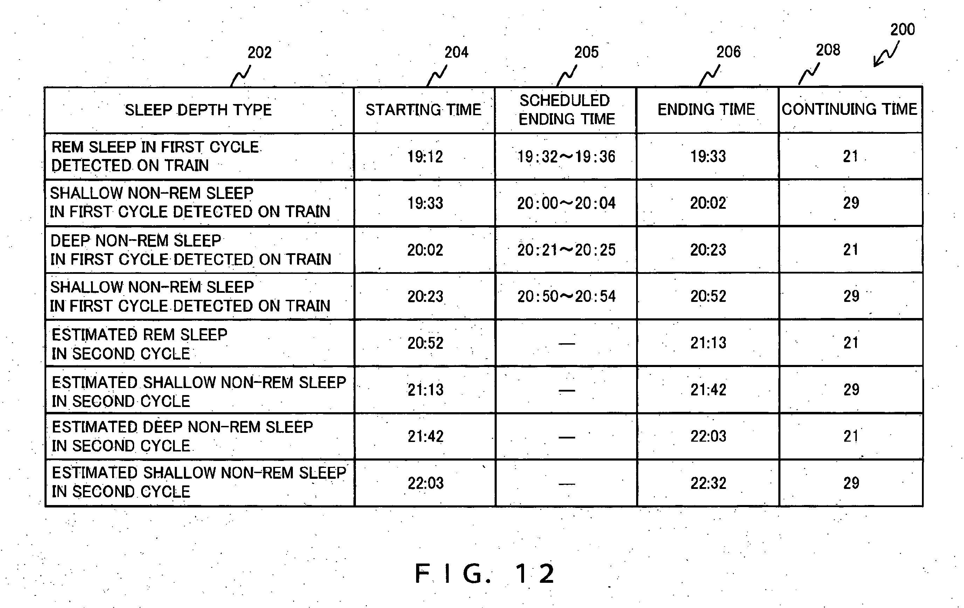

Hereinafter, explanations will be given for a warning issuing method in the mobile terminal 1 according to a second embodiment by referring to FIG. 12 through FIG. 15. In the second embodiment, constituents and operations similar to those in the first embodiment are denoted by the same symbols, and explanations thereof will be omitted. The second embodiment is different in that a sleep depth time estimation table 200 is used instead of the sleep depth time estimation table 100 and different processes are performed in relation to the sleep depth time estimation table 200.

FIG. 12 illustrates an example of the sleep depth time estimation table 200. The sleep depth time estimation table 200 includes a sleep depth type 202, a starting time 204, a scheduled ending time 205, a corrected ending time 206, and a continuing time 208.

The mobile terminal 1 according to the second embodiment measures transitions to sleep depths in one cycle after the detection of boarding. Accordingly, the sleep depth time estimation table 200 includes "shallow non-REM sleep in the first cycle detected on a train", "deep non-REM sleep in the first cycle detected on a train", and "shallow non-REM sleep in the first cycle detected on a train" as the sleep depth type 202. In and after the second cycle, respective types of sleep are estimated types on the basis of the measurement after boarding.

The starting time 204 and the corrected ending time 206 in the first cycle are sleep starting times that were measured in a conveyance. The starting times 204 and the corrected ending times 206 in and after the second cycle are times obtained by sequentially adding the continuing time 208 calculated on the basis of the time measured in the first cycle to the time measured in the first cycle. The scheduled ending time 205 is a scheduled time of sleep depth transition obtained by adding the corresponding continuing time 98 in the sleep depth time management table 90 to the detection time of transition of each sleep depth in the first cycle and considering a prescribed period of time.

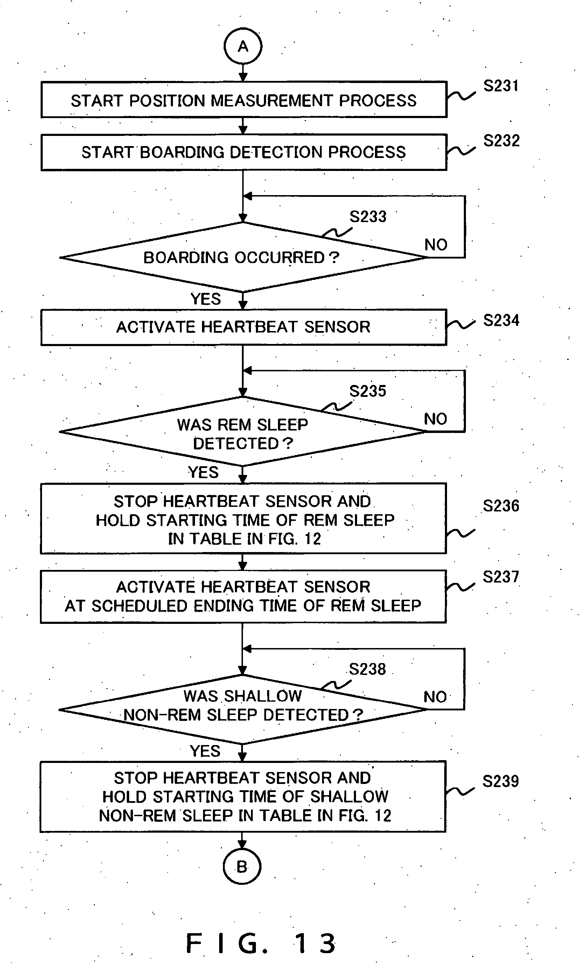

Hereinafter, further explanations will be given for operations of the mobile terminal 1 according to the present embodiment by referring to FIG. 13 through FIG. 15. The processes below performed by the mobile terminal 1 are executed by the CPU 3 by reading a prescribed program from for example the RAM 11 and executing it, however, in the explanations below, it is assumed that they are processes executed by the respective functions explained in FIG. 2.

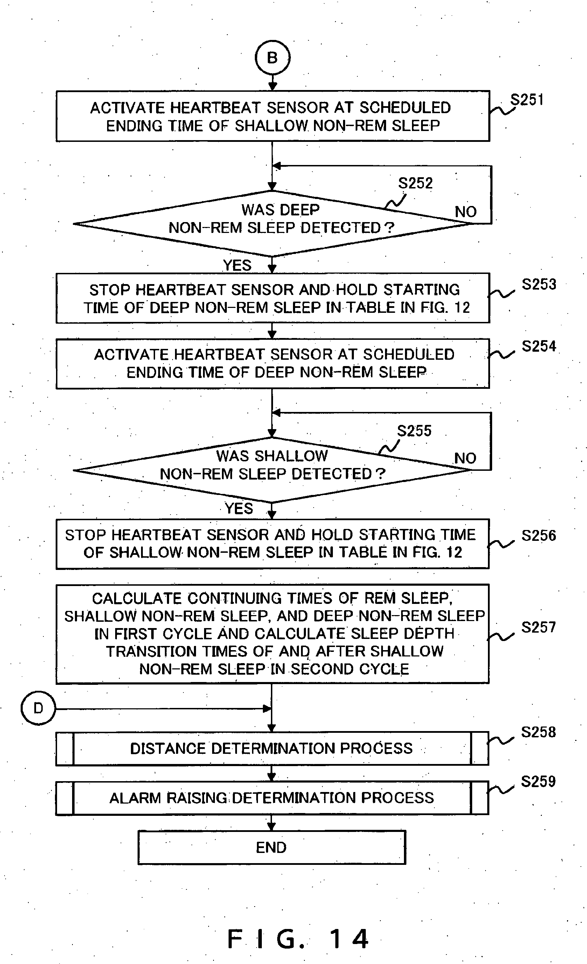

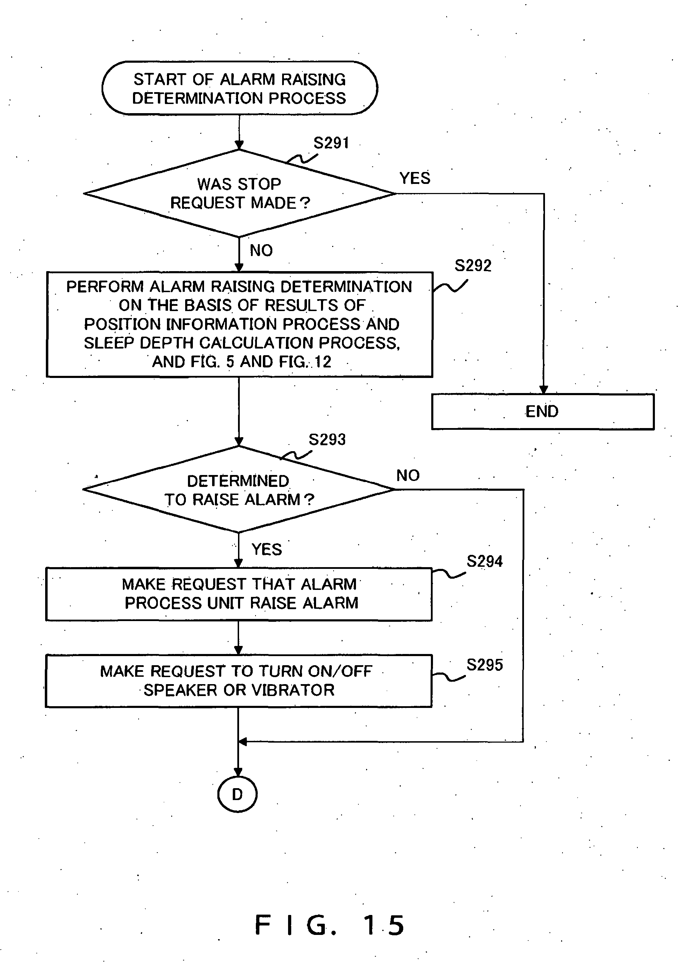

FIG. 13 and FIG. 14 are flowcharts of a warning issuing process of the mobile terminal 1 according to the present embodiment. FIG. 15 is a flowchart of an alarm determination process. In the present embodiment, the mobile terminal 1 first performs the same process as that explained in FIG. 8. Next, as illustrated in FIG. 13, the position information processing unit 53 starts a process of measuring the current position (S231). Specifically, the position information processing unit 53 performs a measuring process for example once in a prescribed period of time by using the GPS 33 so as to obtain the current position. The conveyance detection unit 55 starts the detection of whether or not boarding on a conveyance such as a train occurred (S232). The conveyance detection unit 55 samples acceleration obtained by the acceleration sensor 9 so as to repeat the determination until boarding is detected (NO in S233), and detects whether or not boarding occurred.

When the conveyance detection unit 55 has detected boarding (YES in S233), the sleep depth calculation unit 57 activates the heartbeat sensor 7 (S234). The sleep depth calculation unit 57 repeats determination until detecting REM sleep on the basis of detection value of the heartbeat of the user obtained by the heartbeat sensor 7 (NO in S235). The sleep depth calculation unit 57 stops the heartbeat sensor 7 when it has detected REM sleep (YES in S235), and holds in the sleep depth time estimation table 200 illustrated in FIG. 12 the time at which it detected the sleep (S236) in the starting time 204. Also, it calculates the scheduled ending time 205 of the REM sleep in the sleep depth time estimation table 200. Specifically, the sleep depth calculation unit 57 adds the continuing time 98 of the REM sleep in the sleep depth time management table 90 illustrated in FIG. 6 to the starting time 204 of the detected REM sleep and calculates the scheduled ending time 205 that has been given prescribed time margins such as for example 2 minutes before and after the time. Also, the sleep depth calculation unit 57 activates the heartbeat sensor 7 when the calculated scheduled ending time 205 has arrived.

During the scheduled ending time 205, the sleep depth calculation unit 57 repeats determination until shallow REM sleep is detected on the basis of detection values of the heartbeat of the user obtained by the heartbeat sensor 7 (NO in S238). When the sleep depth calculation unit 57 has detected shallow non-REM sleep (YES in S238), it stops the heartbeat sensor 7, and holds in the sleep depth time estimation table 200 illustrated in FIG. 12 the time of the detection as the starting time 204 of shallow non-REM sleep in the first cycle (S239).

As illustrated in FIG. 14, the sleep depth calculation unit 57 calculates the scheduled ending time 205 of the shallow non-REM sleep in the sleep depth time estimation table 200. Specifically, the sleep depth calculation unit 57 adds the continuing time 98 of REM sleep in the sleep depth time management table 90 illustrated in FIG. 6 to the starting time 204 of the detected shallow non-REM sleep, and calculates the scheduled ending time 205 that has been given prescribed time margins such as for example 2 minutes before and after the time. Also, the sleep depth calculation unit 57 activates the heartbeat sensor 7 when the calculated scheduled ending time 205 has arrived (S251).

During the scheduled ending time 205, the sleep depth calculation unit 57 repeats determination until deep non-REM sleep is detected on the basis of detection values of the heartbeat of the user obtained by the heartbeat sensor 7 (NO in S252). When the sleep depth calculation unit 57 has detected deep non-REM sleep (YES in S252), it stops the heartbeat sensor 7, and holds in the sleep depth time estimation table 200 illustrated in FIG. 12 the time of the detection as the starting time 204 of deep non-REM sleep in the first cycle (S253). Also, the sleep depth calculation unit 57 calculates the scheduled ending time 205 of the deep non-REM sleep in the sleep depth time estimation table 200. Specifically, the sleep depth calculation unit 57 adds the continuing time 98 of the deep non-REM sleep in the sleep depth time management table 90 illustrated in FIG. 6 to the starting time 204 of the detected deep non-REM sleep and calculates the scheduled ending time 205 that has been given prescribed time margins such as for example 2 minutes before and after the time. Also, the sleep depth calculation unit 57 activates the heartbeat sensor 7 when the calculated scheduled ending time 205 has arrived (S254)

During the scheduled ending time 205, the sleep depth calculation unit 57 repeats determination until shallow non-REM sleep is detected on the basis of detection values of the heartbeat of the user obtained by the heartbeat sensor 7 (NO in S255). When the sleep depth calculation unit 57 has detected shallow non-REM sleep (YES in S255), it stops the heartbeat sensor 7, and holds in the sleep depth time estimation table 200 illustrated in FIG. 12 the time of the detection as the starting time 204 of shallow non-REM sleep in the first cycle (S256).

The sleep depth calculation unit 57 calculates each of the continuing times 208 in the first cycle held in the sleep depth time estimation table 200, and sequentially adds the calculated value to the starting time 204 of the last shallow non-REM sleep in the first cycle so as to generate the sleep depth time estimation tables 200 in and after the second cycle (S257). When transitions of sleep depths are not detected, the continuing time 98 held in the sleep depth time management table 90 for example is used.

Next, the position information processing unit 53 performs a distance determination process (S258). Also, the alarm raising determination unit 51 performs an alarm raising determination process (S259). The distance determination process is similar to the process explained in FIG. 10. Similarly to the first embodiment, the position information processing unit 53 reports to the alarm raising determination unit 51 the calculated distance to the destination. The alarm raising determination process will be described in detail below.

As illustrated in FIG. 15, the alarm raising determination unit 51 determines whether or not a stopping request of an application program was made (S291). When a stopping request was made and when an alarm has been raised, the alarm raising determination unit 51 stops the alarm, and the process is terminated (YES in S291). When a stopping request was not made (NO in S291), the alarm raising determination is performed (S292) in accordance with the determination process results by the position information processing unit 53 and the sleep depth calculation unit 57, the alarm table 80 illustrated in FIG. 5, and the sleep depth time estimation table 200 illustrated in FIG. 12. For example, when a distance measured by the position information processing unit 53 and the sleep depths measured by the sleep depth time estimation table 200 are one of states SC, SE, and SF in the alarm table 80, the alarm raising determination unit 51 determines to make the alarm processing unit 59 raise an alarm. In other cases, it determines not to raise an alarm.

In cases where the determination is not a determination to raise an alarm, the alarm raising determination unit 51 makes the process return to S291 (NO in S293). In cases where the determination is a determination to raise an alarm (YES in S293), the alarm raising determination unit 51 makes an alarm raising request to the alarm processing unit 59 (S294). The alarm processing unit 59 makes an ON-OFF request to the speaker 29 or the vibrator 37 (S175), and makes the process return to s258 in FIG. 10.

As described in detail above, according to the mobile terminal 1 of the second embodiment, the sleep depth calculation unit 57 first measures timings of transitions to sleep depths in at least one cycle, stops the heartbeat sensor 7, and generates the sleep depth time management table 90. When the conveyance detection unit 55 has detected boarding, the sleep depth calculation unit 57 drives the heartbeat sensor 7, and when the sleep depth calculation unit 57 has detected the start of REM sleep in the first cycle, it holds the starting time of the REM sleep in the sleep depth time estimation table 200. The sleep depth calculation unit 57 adds the continuing time 98 of the REM sleep detected in the sleep depth time management table 90 to the starting time of the REM sleep so as to calculate the scheduled ending time 205 of the REM sleep.

The sleep depth calculation unit 57 activates the heartbeat sensor 7 when the scheduled ending time 205 has arrived, and when it has detected the start of shallow non-REM sleep, it stops the heartbeat sensor 7 and holds the time of the detection in the sleep depth time estimation table 200. Similarly, it calculates the scheduled ending time 205 so as to activate the heartbeat sensor 7, and detects the respective transitions to deep non-REM sleep, and shallow non-REM sleep in the first cycle. The sleep depth calculation unit 57 calculates the respective times in and after the second cycle in the sleep depth time estimation table 200 on the basis of the detected transition times. Also, the sleep depth calculation unit 57 stops the heartbeat sensor 7 each time a transition to a sleep depth is detected. The position information processing unit 53 obtains the current position by using the GPS 33 for example once in a prescribed period of time, and calculates the distance to the destination that has been obtained beforehand.

The alarm raising determination unit 51 determines whether or not the calculated distance and a sleep depth estimated by the sleep depth time estimation table 200 meet a prescribed condition in the alarm table 80. When they do, the alarm raising determination unit 51 issues a warning by using the alarm processing unit 59. Specifically, the alarm raising determination unit 51 issues a warning regardless of a sleep depth when the distance from the destination is shorter than threshold Pc. Also, when the distance from the destination is shorter than Pb, which is greater than threshold Pc, and when it has been determined that a sleep depth is shallow, a warning is issued.

As described above, in the warning issuing method in the mobile terminal 1 according to the second embodiment, the effects as below will be attained in addition to the effects attained by the first embodiment. Specifically, actual transitions to sleep depths are measured for one cycle after boarding so that sleep depths are predicted on the basis of measured timings, making it possible to determine sleep more accurately. Accordingly, it is possible to further reduce cases where a user does not notice warnings. Also, the heartbeat sensor 7 is not driven before boarding is detected or until a scheduled time of the transition to a next sleep depth after the detection of a transition of a sleep depth. Accordingly, it is not necessary to keep the heartbeat sensor 7 driven continuously. This makes it possible to suppress increases in power consumption while predicting transitions to sleep depths more accurately.

### (Third Embodiment)

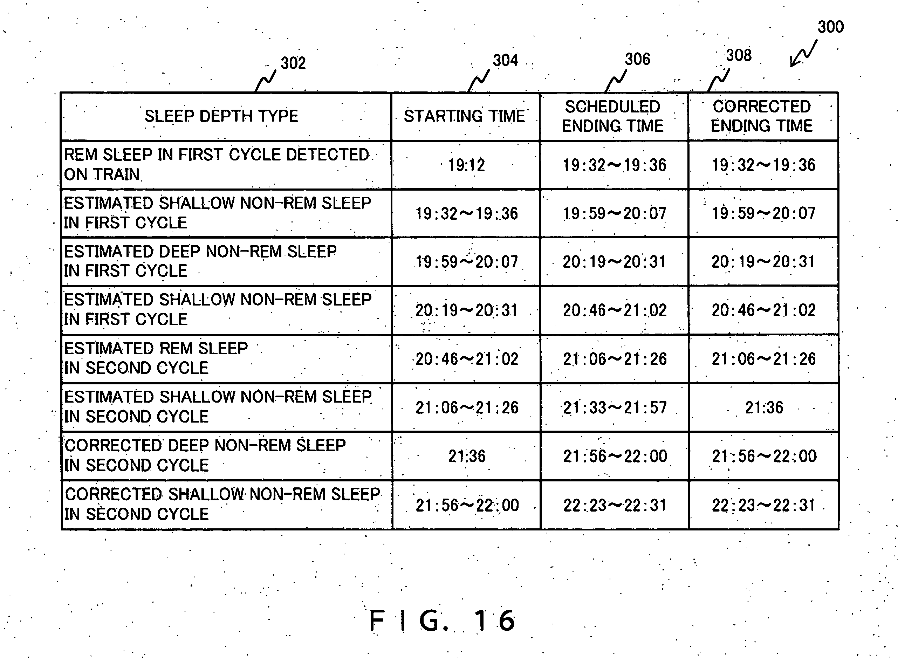

Hereinafter, by referring to FIG. 16 through FIG. 18, a warning issuing method in the mobile terminal 1 according to a third embodiment will be explained. In the third embodiment, constituents similar to those in the mobile terminal 1 according to the first or the second embodiment are denoted by the same symbols, and explanations thereof will be omitted. The third embodiment is different in that a sleep depth time estimation table 300 is used instead of the sleep depth time estimation table 100 or the sleep depth time estimation table 200 and different processes are performed in relation to the sleep depth time estimation table 300.

FIG. 16 illustrates an example of the sleep depth time estimation table 300. As illustrated in FIG. 16, the sleep depth time estimation table 300 includes a sleep depth type 302, a starting time 304, a scheduled ending time 306, and a corrected ending time 308. The starting time 304 of "REM sleep in the first cycle detected on a train" is a time of actual detection after boarding. The scheduled ending time 306 of "REM sleep in the first cycle detected on a train" is a period of time obtained by adding the continuing time 98 of REM sleep in the sleep depth time management table 90 illustrated in FIG. 6 to the starting time 304 and giving prescribed time margins such as for example two minutes before and after that period. The corrected ending time 308 is a time of detection when a transition to a sleep depth has been detected during the scheduled ending time 306, and is the same as the scheduled ending time 306 when no transitions have been detected.

The starting time 304 in and after the shallow non-REM sleep in the first cycle is the same as the corrected ending time 308 of the previous sleep depth type 302. The scheduled ending time 306 in and after the shallow non-REM sleep in the first cycle is calculated as below. Specifically, the earlier time between the scheduled ending times 306 is a time obtained by adding the corresponding continuing time 98 to the earliest time as the corresponding starting time 304 and putting the time ahead by a prescribed period of time. The later time between the scheduled ending times 306 is a time obtained by adding the corresponding continuing time 98 to the latest time as the starting time 304 and putting the time back by a prescribed period of time.

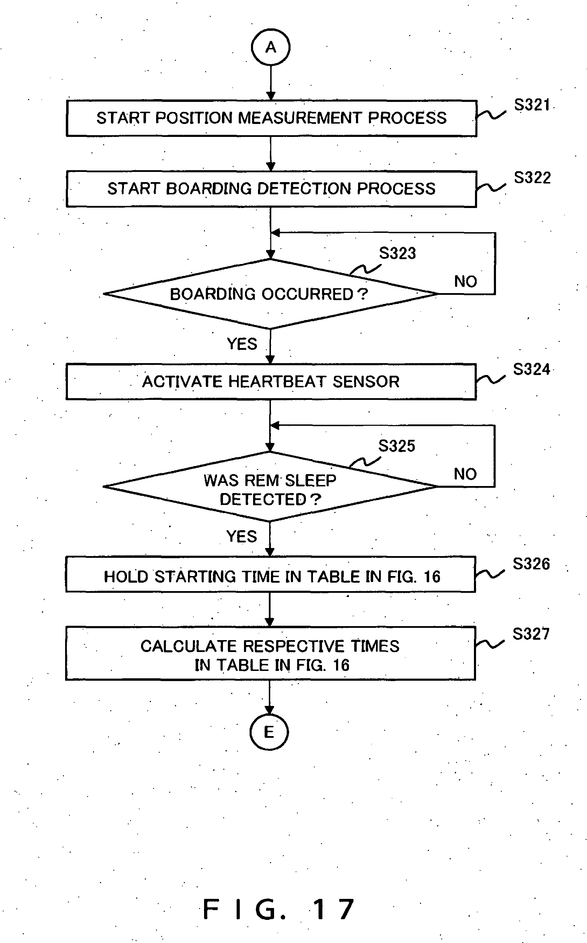

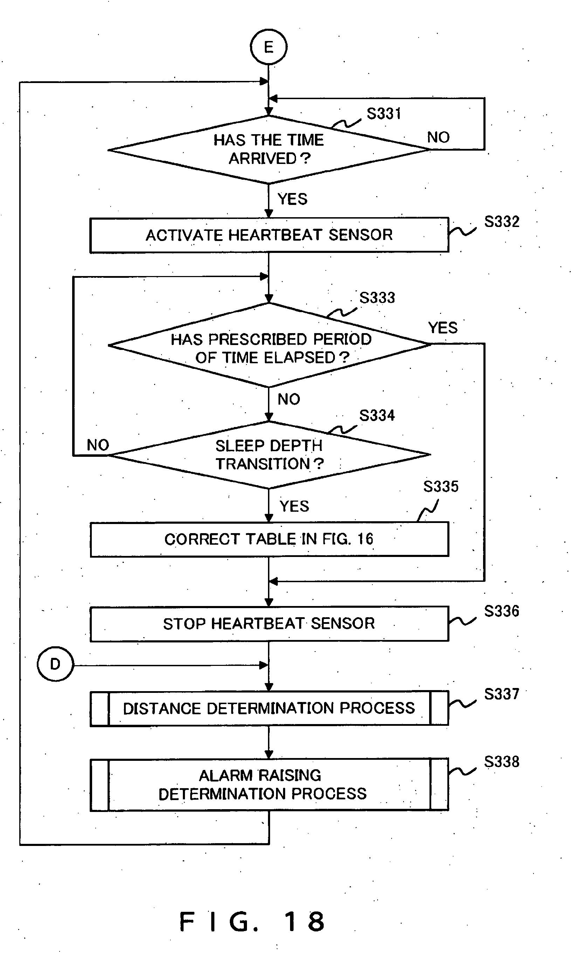

Hereinafter, by referring to FIG. 17 and FIG. 18, the warning issuing method according to the third embodiment will be explained further. The processes below performed by the mobile terminal 1 are implemented by the application CPU 3 executing a prescribed program after reading it from for example the RAM 11. However, explanations will be given on an assumption that the processes are executed by the respective functions explained with reference to FIG. 2.

FIG. 17 and FIG. 18 are flowcharts illustrating the warning issuing method in the mobile terminal 1 according to the present embodiment. In the present embodiment, the mobile terminal 1 first performs processes similar to those explained by referring to FIG. 8.

As illustrated in FIG. 17, the position information processing unit 53 starts a measurement process of the current position. Specifically, the position information processing unit 53 obtains the current position by performing a measurement process for example once in a prescribed period of time by using the GPS 33. The conveyance detection unit 55 starts the detection of whether or not boarding on a conveyance such as a train or the like occurred (S322). The conveyance detection unit 55 samples acceleration obtained by the acceleration sensor 9 so as to repeat the determination until boarding is detected (NO in S323). Whether or not boarding on a conveyance occurred can be performed by using a known technique such as for example a method described in non-Patent Document 2 or the like.

When the conveyance detection unit 55 has detected boarding (YES in S323), the sleep depth calculation unit 57 activates the heartbeat sensor 7 (S324). The sleep depth calculation unit 57 repeats determination until detecting REM sleep on the basis of detection values of the heartbeat of the user obtained by the heartbeat sensor 7 (NO in S325). When the sleep depth calculation unit 57 has detected REM sleep (YES in S325), it stops the heartbeat sensor 7, and holds in the sleep depth time estimation table 300 illustrated in FIG. 12 the time of the detection as the starting time 304 of REM sleep in the first cycle (S326). Also, the sleep depth calculation unit 57 calculates prediction of the time of each transition to a sleep depth in the sleep depth time estimation table 300 on the basis of the sleep depth time management table 90 illustrated in FIG. 6 (S327).

For example, the sleep depth calculation unit 57 calculates a period of time obtained by adding the continuing time 98 (22 minutes) corresponding in the sleep depth time management table 90 to the starting time (19 : 12) of "REM sleep in the first cycle detected on a train" that has been given prescribed time margins. In the example illustrated in FIG. 16, a period of time that has been given ±2 minutes (19:32 through 19:36) is treated as the scheduled ending time 306 of the REM sleep in the first cycle. The corrected ending time 308 and the starting time 304 of the next sleep depth type 302 are same values as the scheduled ending time 306.

During the next "estimated shallow non-REM sleep in the first cycle", the continuing time 98 (29 minutes) of the shallow non-REM sleep is added to the earliest time (19:32) of the starting time 304 as the earliest time of the scheduled ending time 306 and two minutes is subtracted (19:59). As the latest time of the scheduled ending time 306, the continuing time 98 (29 minutes) of the shallow non-REM sleep is added to the latest time (19:36) of the starting time 304 and two minutes is added. (20:07). Treating this period of time as the scheduled ending time 306, the sleep depth calculation unit 57 drives the heartbeat sensor 7. The sleep depth calculation unit 57 repeats this process so as to calculate the respective times in the sleep depth time estimation table 300.

As illustrated in FIG. 18, the sleep depth calculation unit 57 refers to the sleep depth time estimation table 300 and repeats the detection of times until the corrected ending time 308 of the next sleep depth arrives (NO in S331). When the time has arrived (YES in S331), the sleep depth calculation unit 57 activates the heartbeat sensor 7 (S332), and determines whether or not the corrected ending time 308 has elapsed (S333) . When it has been determined that the corrected ending time 308 has elapsed (YES in S333), the sleep depth calculation unit 57 makes the process proceed to S336.

When the corrected ending time 308 has not elapsed (NO in S333), the sleep depth calculation unit 57 determines whether or not a transition to a sleep depth has been detected (S334). When no transitions to a sleep depth have been detected (NO in S334), the sleep depth calculation unit 57 makes the process return to S333 so as to repeat the process.

When a transition to a sleep depth has been detected (YES in S334), the sleep depth calculation unit 57 updates the corresponding 308 in the sleep depth time estimation table 300 illustrated in FIG. 16 to the time at which a transition to a sleep depth has been detected (S335), and stops the heartbeat sensor 7 (S336).

For example, it is assumed in FIG. 16 that a transition to deep non-REM sleep next to the "estimated shallow non-REM sleep in the estimated second cycle" has been detected at 21:36. The sleep depth calculation unit 57 updates the starting time 304 of the next "deep non-REM sleep in the corrected second cycle" to "21:36", and calculates the respective times after that time in the sleep depth time estimation table 300 in a manner similar to S327, and performs modification. Also, this process is repeated. When a prescribed period of time has elapsed without detecting a transition to a sleep state, the corrected ending time 308 is held as it is the scheduled ending time 306.

Next, the position information processing unit 53 performs a distance determination process (S337). Also, the alarm raising determination unit 51 performs an alarm raising determination process (S338). The distance determination process is similar to the process explained in the first or second embodiment. Also, similarly to the first or second embodiment, the position information processing unit 53 reports to the alarm raising determination unit 51 the calculated distance to the destination.

The alarm raising determination process is similar in detailed aspects to the alarm raising determination process according to the first or second embodiment. For example, in S172, explained in the first embodiment, a sleep depth time estimation table 300 is used instead of the sleep depth time estimation table 100, which is a different point. Also, it is possible to determine only a time not including the predicted starting time 304 or the corrected ending time 308 as being "shallow" or "deep". After the termination of the alarm raising determination process, the process returns to S337 illustrated in FIG. 18.

As described in detail above, according to the mobile terminal 1 of the third embodiment, the sleep depth calculation unit 57 first measures timings of transitions to sleep depths in at least one cycle, stops the heartbeat sensor 7, and generates the sleep depth time management table 90. When the conveyance detection unit 55 has detected boarding, the sleep depth calculation unit 57 drives the heartbeat sensor 7. When the sleep depth calculation unit 57 has detected the start of REM sleep in the first cycle, it holds the starting time of the REM sleep in the sleep depth time estimation table 300 on the basis of the starting time and the sleep depth time management table 90. The sleep depth calculation unit 57 adds the continuing time 98 of the REM sleep detected in the sleep depth time management table 90 to the starting time of the REM sleep and gives time margins to the calculated time so as to calculate the scheduled ending time 306 of the REM sleep.

When the sleep depth calculation unit 57 detects the transition to the next sleep depth after activating the heartbeat sensor 7 upon the start of the scheduled ending time 306, it stops the heartbeat sensor 7, and holds the detection time as the corrected ending time 308 in the sleep depth time estimation table 300. Also, it calculates and updates the scheduled ending time 306 and the corrected ending time 308 from the next sleep depth type 302 in the sleep depth time estimation table 300. When no transitions have been detected, it stops the heartbeat sensor 7 at the end of the corrected ending time 308.

The position information processing unit 53 obtains the current position by using the GPS 33 for example once in a prescribed period of time, and calculates the distance to the destination that has been obtained beforehand. The alarm raising determination unit 51 determines whether or not the calculated distance and a sleep depth estimated by the sleep depth time estimation table 200 meet a prescribed condition in the alarm table 80. When they do, the alarm raising determination unit 51 issues a warning by using the alarm processing unit 59. Specifically, the alarm raising determination unit 51 issues a warning regardless of a sleep depth when the distance from the destination is shorter than threshold Pc. Also, when the distance from the destination is shorter than Pb, which is greater than threshold Pc, and when it has been determined that a sleep depth is shallow, a warning is issued.

As described above, in the warning issuing method in the mobile terminal 1 according to the third embodiment, the effects as below will be attained in addition to the effects attained by the first or second embodiment. Specifically, actual transitions to sleep depths are measured for one cycle after boarding so that sleep depths are predicted on the basis of measured timings, making it possible to determine sleep more accurately. Upon this, the heartbeat sensor 7 is driven only at the scheduled ending time 306 so that power saving is expected. Also, time margins are given to time for activating the heartbeat sensor 7, making it possible to detect transitions of sleep more reliably. Accordingly, it is possible to further reduce cases where a user does not notice warnings.

In the mobile terminal 1 according to the present embodiment, the heartbeat sensor 7 is not driven before the detection of boarding or except in periods of time for detecting transitions to sleep depths. Accordingly, it is not necessary to keep the heartbeat sensor 7 driven continuously. This makes it possible to suppress increases in power consumption while predicting transitions to sleep depths more accurately.

As described above, an information processing apparatus, a warning issuing method, and a storage medium having stored therein a program that can reduce cases where warnings are not noticed by avoiding a case of insufficient amount of power caused by sleep depth measurement when it is to be made known that destinations have gotten closer.

In the first through third embodiments, the GPS 33 is an example of the position information detection device, while the speaker 29 and the vibrator 37 are examples of the warning issuing device. The application CPU 3 is an example of a processor.

Note that the scope of the present invention is not limited to the above described embodiments, and various configurations or embodiments can be employed without departing from the scope of the claims. For example, the configuration of the mobile terminal 1 is not limited to the examples explained in FIG. 1 or FIG. 2, and may be a different configuration that can perform the substantially same processes. The process of display of the display example 65 (S122) that is common across the first through third embodiments may be omitted at that moment. The position information processing unit 53 may display the display example 65 after the detection of boarding.

The alarm table 80 is an example, and alterations are allowed as long as it performs substantially similar determination. Also, in the above examples, the sleep depth time management table 90 is generated on the basis of sleep for one cycle in the day previous to boarding, however, it is also possible to store and utilize general sleep examples beforehand. It is also possible to recognize that "shallow" sleep depth type 86 includes not only REM sleep as above but also shallow non-REM sleep. In such a case, "shallow" is discriminated from deep non-REM sleep. The above first through third embodiments may be combined arbitrarily in all feasible manners.

## Claims

1. An information processing apparatus comprising:
a heartbeat sensor (7) configured to measure heartbeat of a user;
an acceleration sensor (9) configured to detect acceleration;
a position information detection device (33) configured to detect position information;
a warning issuing device (29, 37) configured to issue a warning;
a storage device (11) configured to hold a first cycle based on transition times of sleep depths for one cycle; and
a processor (3) configured to detect boarding of the user on a conveyance on the basis of the acceleration, to activate the heartbeat sensor when the boarding has been detected, to detect a transition to sleep of the user and a transition time to the sleep on the basis of heartbeat measured by the heartbeat sensor, to stop driving of the heartbeat sensor when the transition has been detected, and to make the warning issuing device issue a warning when it has been determined that a distance between a position represented by the position information detected by the position information detection device and a destination is shorter than a prescribed value and that a sleep depth of the user is shallow on the basis of the first cycle and a transition time to the sleep.

2. The information processing apparatus according to claim 1, wherein:
the processor (3) further obtains a starting instruction of measurement for calculating the first cycle, activates the heartbeat sensor after obtaining the starting instruction, performs measurement by the heartbeat sensor, and calculates the first cycle on the basis of a result of the measurement.

3. The information processing apparatus according to claim 1 or 2, wherein:
the processor (3) drives the heartbeat sensor intermittently in accordance with a time of a transition to a sleep depth predicted on the basis of a time of detection of a transition to the sleep depth and the first cycle until transitions to the sleep depths are detected for one cycle after detection of a transition to the sleep after the boarding detection, calculates a second cycle on the basis of measurement values for the detected one cycle, and issues the warning when it has been determined that a distance between a position represented by the position information detected by the position information detection device and a destination is shorter than a prescribed value and that a sleep depth of the user is shallow on the basis of the second cycle and a transition time to the sleep.

4. The information processing apparatus according to claim 1 or 2, wherein:
the processor (3) drives the heartbeat sensor during a period of time between an earliest time and a latest time of predicted transition times to sleep depths calculated on the basis of a time at which the boarding was detected, the first cycle, and a variation predicted for the first cycle, modifies a predicted transition time of the sleep depth on the basis of the detection time when a transition to the sleep depth has been detected, and issues the warning when it has been determined that a distance between a position represented by the position information detected by the position information detection device and a destination is shorter than a prescribed value and that a sleep depth of the user is shallow on the basis of the detection time and a transition time to the sleep.

5. The information processing apparatus according to claim4, wherein:
when a transition to the sleep depth is not detected before a latest time of the predicted transition times, a period of time of a predicted transition time corresponding to a transition to a next sleep depth is between a time obtained by adding, to the earliest time, a time obtained by reducing a margin of the variation from a time based on the first cycle and a time obtained by adding, to the latest time, a time obtained by adding a margin of the variation to a time based on the first cycle.

6. A warning issuing method, comprising:
detecting boarding of a user on a conveyance on the basis of acceleration detected by an acceleration sensor (9);
activating a heartbeat sensor (7);
detecting a transition to sleep of the user on the basis of heartbeat measured by the heartbeat sensor (7);
stopping driving of the heartbeat sensor (7);
determining whether or not conditions that a distance between a position represented by position information detected by a position information detection device (33) and a destination is shorter than a prescribed value and that a sleep depth of the user is shallow on the basis of a first cycle based on transition times of sleep depths for one cycle and a transition time to the sleep are met by a processor; and
issues a warning when the conditions are met.

7. The warning issuing method according to claim 6, further comprising:
performing measurement by the heartbeat sensor by activating the heartbeat sensor when a starting instruction of measurement for calculating the first cycle has been obtained; and
calculating the first cycle on the basis of a result of the measurement by the processor.

8. The warning issuing method according to claim 6 or 7, further comprising:
detecting a transition to the sleep state after the boarding detection;
driving the heartbeat sensor intermittently in accordance with a time of a transition to a sleep depth predicted on the basis of a time of detection of a transition to the sleep depth and the first cycle until transitions to the sleep depths are detected for one cycle;
calculating a second cycle on the basis of measurement values for the detected one cycle; and
issuing the warning when it has been determined that a distance between a position represented by the position information detected by the position information detection device and a destination is shorter than a prescribed value and that a sleep depth of the user is shallow on the basis of the second cycle and a transition time to the sleep by the processor.

9. The warning issuing method according to claim 6 or 7, further comprising:
driving the heartbeat sensor during a period of time between an earliest time and a latest time of predicted transition times to sleep depths calculated on the basis of a time at which the boarding was detected, the first cycle, and a variation predicted for the first cycle;
detecting a transition to the sleep depth;
modifying a predicted transition time of the sleep depth on the basis of the detection time; and
issuing the warning when it has been determined that a distance between a position represented by the position information detected by the position information detection device and a destination is shorter than a prescribed value and that a sleep depth of the user is shallow on the basis of the detection time and a transition time to the sleep by the processor.

10. The warning issuing method according to claim 9, wherein:
when a transition to the sleep depth is not detected before a latest time of the predicted transition times, a period of time of a predicted transition time corresponding to a transition to a next sleep depth is between a time obtained by adding, to the earliest time, a time obtained by reducing a margin of the variation from a time based on the first cycle and a time obtained by adding, to the latest time, a time obtained by adding a margin of the variation to a time based on the first cycle.

11. A program for causing a computer to execute a process comprising:
detecting boarding of a user on a conveyance on the basis of acceleration detected by an acceleration sensor (9);
activating a heartbeat sensor (7);
detecting a transition to sleep of the user on the basis of heartbeat measured by the heartbeat sensor (7);
stopping driving of the heartbeat sensor (7);
determining whether or not conditions that a distance between a position represented by position information detected by a position information detection device (33) and a destination is shorter than a prescribed value and that a sleep depth of the user is shallow on the basis of a first cycle based on transition times of sleep depths for one cycle and a transition time to the sleep are met; and
issuing a warning when the conditions are met.

12. The program according to claim 11, the process further comprising:
performing measurement by the heartbeat sensor by activating the heartbeat sensor when a starting instruction of measurement for calculating the first cycle has been obtained; and
calculating the first cycle on the basis of a result of the measurement.

13. The program according to claim 11 or 12, the process further-comprising:
detecting a transition to the sleep state after the boarding detection;
driving the heartbeat sensor intermittently in accordance with a time of a transition to a sleep depth predicted on the basis of a time of detection of a transition to the sleep depth and the first cycle until transitions to the sleep depths are detected for one cycle;
calculating a second cycle on the basis of measurement values for the detected one cycle; and
issuing the warning when it has been determined that a distance between a position represented by the position information detected by the position information detection device and a destination is shorter than a prescribed value and that a sleep depth of the user is shallow on the basis of the second cycle and a transition time to the sleep.

14. The program according to claim 11 or 12, the process further comprising:
driving the heartbeat sensor during a period of time between an earliest time and a latest time of predicted transition times to sleep depths calculated on the basis of a time at which the boarding was detected, the first cycle, and a variation predicted for the first cycle;
detecting a transition to the sleep depth;
modifying a predicted transition time of the sleep depth on the basis of the detection time; and
issuing the warning when it has been determined that a distance between a position represented by the position information detected by the position information detection device and a destination is shorter than a prescribed value and that a sleep depth of the user is shallow on the basis of the detection time and a transition time to the sleep.

15. The program according to claim 14, wherein:
when a transition to the sleep depth is not detected before a latest time of the predicted transition times, a period of time of a predicted transition time corresponding to a transition to a next sleep depth is between a time obtained by adding, to the earliest time, a time obtained by reducing a margin of the variation from a time based on the first cycle and a time obtained by adding, to the latest time, a time obtained by adding a margin of the variation to a time based on the first cycle.

## Patentansprüche

1. Informationsverarbeitungsvorrichtung, umfassend:
einen Herzschlagsensor (7), der eingerichtet ist zum Messen eines Herzschlags eines Benutzers;
einen Beschleunigungssensor (9), der eingerichtet ist zum Messen einer Beschleunigung;
eine Positionsinformations-Erfassungseinrichtung (33), die eingerichtet ist zum Erfassen von Positionsinformation;
eine Warnungsausgabeeinrichtung (29, 37), die eingerichtet ist zum Ausgeben einer Warnung;
eine Speichereinrichtung (11), die eingerichtet ist zum Halten eines Zyklus basierend auf Übergangszeiten von Schlaftiefen für einen Zyklus; und
einen Prozessor (3), der eingerichtet ist zum Erfassen eines Einsteigens des Benutzers in ein Transportmittel auf Basis der Beschleunigung, zum Aktivieren des Herzschlagsensors, wenn das Einsteigen erfasst wurde, zum Erfassen eines Übergangs in einen Schlaf des Benutzers und einer Übergangszeit in den Schlaf basierend auf dem von dem Herzschlagsensor gemessenen Herzschlag, zum Stoppen eines Antreibens des Herzschlagsensors, wenn der Übergang erfasst wurde, und zum Veranlassen der Warnungsausgabeeinrichtung, eine Warnung auszugeben, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis des ersten Zyklus und einer Übergangszeit in den Schlaf.

2. Informationsverarbeitungsvorrichtung nach Anspruch 1, wobei:
der Prozessor (3) weiterhin einen Startbefehl einer Messung zum Berechnen des ersten Zyklus erhält, den Herzschlagsensor nach Erhalt des Startbefehls aktiviert, eine Messung durch den Herzschlagsensor durchführt und den ersten Zyklus basierend auf einem Ergebnis der Messung berechnet.

3. Informationsverarbeitungsvorrichtung nach Anspruch 1 oder 2, wobei:
der Prozessor (3) den Herzschlagsensor intermittierend antreibt in Übereinstimmung mit einer Zeit eines Übergangs in eine Schlaftiefe, die vorhergesagt wird auf Basis einer Erfassungszeit eines Übergangs in die Schlaftiefe und dem ersten Zyklus bis Übergänge in die Schlaftiefen erfasst werden für einen Zyklus nach einer Erfassung eines Übergangs in den Schlaf nach der Einstiegserfassung, einen zweiten Zyklus auf Basis von Messwerten für den erfassten einen Zyklus berechnet, und die Warnung ausgibt, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis des zweiten Zyklus und einer Übergangszeit in den Schlaf.

4. Informationsverarbeitungsvorrichtung nach Anspruch 1 oder 2, wobei:
der Prozessor (3) den Herzschlagsensor antreibt während einer Zeitperiode zwischen einer frühsten Zeit und einer spätesten Zeit von vorhergesagten Übergangszeiten in Schlaftiefen, die berechnet werden auf Basis einer Zeit, zu der das Einsteigen erfasst wurde, dem ersten Zyklus und einer für den ersten Zyklus vorhergesagten Variation, eine vorhergesagte Übergangszeit der Schlaftiefe modifiziert auf Basis der Erfassungszeit, wenn ein Übergang in die Schlaftiefe erfasst wurde, und die Warnung ausgibt, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis der Erfassungszeit und einer Übergangszeit in den Schlaf.

5. Informationsverarbeitungsvorrichtung nach Anspruch 4, wobei:
wenn ein Übergang in die Schlaftiefe nicht vor einer spätesten Zeit der vorhergesagten Übergangszeiten erfasst wird, eine Zeitperiode einer vorhergesagten Übergangszeit entsprechend einem Übergang in eine nächste Schlaftiefe zwischen einer Zeit, die erhalten wird durch Addieren, zu der frühsten Zeit, einer Zeit, die erhalten wird durch Reduzieren eines Bereichs von der Variation von einer Zeit basierend auf dem ersten Zyklus, und einer Zeit ist, die erhalten wird durch Addieren, zu der spätesten Zeit, einer Zeit, die erhalten wird durch Addieren eines Bereichs der Variation zu einer Zeit basierend auf dem ersten Zyklus.

6. Warnungsausgabeverfahren, umfassend:
Erfassen eines Einsteigens eines Benutzers in ein Transportmittel auf Basis einer von einem Beschleunigungssensor (9) erfassten Beschleunigung;
Aktivieren eines Herzschlagsensors (7);
Erfassen eines Übergangs in einen Schlaf des Benutzers auf Basis des von dem Herzschlagsensor (7) gemessenen Herzschlags;
Stoppen eines Antreibens des Herzschlagsensors (7);
Bestimmen durch einen Prozessor, ob Bedingungen eintreten oder nicht, dass eine Distanz zwischen einer Position, die von einer durch die Positionsinformations-Erfassungseinrichtung (33) erfassten Positionsinformation dargestellt wird, und einem Ziel kürzer ist als ein vorgeschriebener Wert, und dass eine Schlaftiefe des Benutzers flach ist, auf Basis des ersten Zyklus und einer Übergangszeit in den Schlaf; und
Ausgeben einer Warnung, wenn die Bedingungen eintreten.

7. Warnungsausgabeverfahren nach Anspruch 6, weiterhin umfassend:
Durchführen einer Messung durch den Herzschlagsensor durch Aktivieren des Herzschlagsensors, wenn ein Startbefehl einer Messung zum Berechnen des ersten Zyklus erhalten wurde; und
Berechnen des ersten Zyklus auf Basis eines Ergebnisses der Messung durch den Prozessor.

8. Warnungsausgabeverfahren nach Anspruch 6 oder 7, weiterhin umfassend:
Erfassen eines Übergangs in den Schlafzustand nach der Einstiegserfassung;
Antreiben des Herzschlagsensors intermittierend in Übereinstimmung mit einer Zeit eines Übergangs in eine Schlaftiefe, die vorhergesagt wird auf Basis einer Erfassungszeit eines Übergangs in die Schlaftiefe und dem ersten Zyklus bis Übergänge in die Schlaftiefen erfasst werden für einen Zyklus;
Berechnen eines zweiten Zyklus auf Basis von Messwerten für den erfassten einen Zyklus;
Ausgeben der Warnung, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis des zweiten Zyklus und einer Übergangszeit in den Schlaf.

9. Warnungsausgabe Verfahren nach Anspruch 6 oder 7, weiterhin umfassend:
Antreiben des Herzschlagsensors während einer Zeitperiode zwischen einer frühsten Zeit und einer spätesten Zeit von vorhergesagten Übergangszeiten in Schlaftiefen, die berechnet werden auf Basis einer Zeit, zu der das Einsteigen erfasst wurde, dem ersten Zyklus und einer für den ersten Zyklus vorhergesagten Variation;
Erfassen eines Übergangs in die Schlaftiefe;
Modifizieren einer vorhergesagten Übergangszeit der Schlaftiefe auf Basis der Erfassungszeit; und Ausgeben der Warnung, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis der Erfassungszeit und einer Übergangszeit in den Schlaf.

10. Warnungsausgabe Verfahren nach Anspruch 9, wobei:
wenn ein Übergang in die Schlaftiefe nicht vor einer spätesten Zeit der vorhergesagten Übergangszeiten erfasst wird, eine Zeitperiode einer vorhergesagten Übergangszeit entsprechend einem Übergang in eine nächste Schlaftiefe zwischen einer Zeit, die erhalten wird durch Addieren, zu der frühsten Zeit, einer Zeit, die erhalten wird durch Reduzieren eines Bereichs von der Variation von einer Zeit basierend auf dem ersten Zyklus, und einer Zeit ist, die erhalten wird durch Addieren, zu der spätesten Zeit, einer Zeit, die erhalten wird durch Addieren eines Bereichs der Variation zu einer Zeit basierend auf dem ersten Zyklus.

11. Programm, das einen Computer veranlasst, einen Prozess auszuführen, der umfasst:
Erfassen eines Einsteigens eines Benutzers in ein Transportmittel auf Basis einer von einem Beschleunigungssensor (9) erfassten Beschleunigung;
Aktivieren eines Herzschlagsensors (7);
Erfassen eines Übergangs in einen Schlaf des Benutzers auf Basis des von dem Herzschlagsensor (7) gemessenen Herzschlags;
Stoppen eines Antreibens des Herzschlagsensors (7);
Bestimmen, ob Bedingungen eintreten oder nicht, dass eine Distanz zwischen einer Position, die von einer durch die Positionsinformations-Erfassungseinrichtung (33) erfassten Positionsinformation dargestellt wird, und einem Ziel kürzer ist als ein vorgeschriebener Wert, und dass eine Schlaftiefe des Benutzers flach ist, auf Basis des ersten Zyklus und einer Übergangszeit in den Schlaf; und
Ausgeben einer Warnung, wenn die Bedingungen eintreten.

12. Programm nach Anspruch 11, wobei der Prozess weiterhin umfasst:
Durchführen einer Messung durch den Herzschlagsensor durch Aktivieren des Herzschlagsensors, wenn ein Startbefehl einer Messung zum Berechnen des ersten Zyklus erhalten wurde; und
Berechnen des ersten Zyklus auf Basis eines Ergebnisses der Messung.

13. Programm nach Anspruch 11 oder 12, wobei der Prozess weiterhin umfasst:
Erfassen eines Übergangs in den Schlafzustand nach der Einstiegserfassung;
Antreiben des Herzschlagsensors intermittierend in Übereinstimmung mit einer Zeit eines Übergangs in eine Schlaftiefe, die vorhergesagt wird auf Basis einer Erfassungszeit eines Übergangs in die Schlaftiefe und dem ersten Zyklus bis Übergänge in die Schlaftiefen erfasst werden für einen Zyklus;
Berechnen eines zweiten Zyklus auf Basis von Messwerten für den erfassten einen Zyklus;
Ausgeben der Warnung, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis des zweiten Zyklus und einer Übergangszeit in den Schlaf.

14. Programm nach Anspruch 11 oder 12, wobei der Prozess weiterhin umfasst:
Antreiben des Herzschlagsensors während einer Zeitperiode zwischen einer frühsten Zeit und einer spätesten Zeit von vorhergesagten Übergangszeiten in Schlaftiefen, die berechnet werden auf Basis einer Zeit, zu der das Einsteigen erfasst wurde, dem ersten Zyklus und einer für den ersten Zyklus vorhergesagten Variation;
Erfassen eines Übergangs in die Schlaftiefe;
Modifizieren einer vorhergesagten Übergangszeit der Schlaftiefe auf Basis der Erfassungszeit; und
Ausgeben der Warnung, wenn bestimmt wurde, dass eine Distanz zwischen einer Position, die von der durch die Positionsinformations-Erfassungseinrichtung erfassten Positionsinformation dargestellt wird, und einem Ziel kürzer ist als ein vorgeschriebener Wert, und dass eine Schlaftiefe des Benutzers flach ist, auf Basis der Erfassungszeit und einer Übergangszeit in den Schlaf.

15. Programm nach Anspruch 14, wobei:
wenn ein Übergang in die Schlaftiefe nicht vor einer spätesten Zeit der vorhergesagten Übergangszeiten erfasst wird, eine Zeitperiode einer vorhergesagten Übergangszeit entsprechend einem Übergang in eine nächste Schlaftiefe zwischen einer Zeit, die erhalten wird durch Addieren, zu der frühsten Zeit, einer Zeit, die erhalten wird durch Reduzieren eines Bereichs von der Variation von einer Zeit basierend auf dem ersten Zyklus, und einer Zeit ist, die erhalten wird durch Addieren, zu der spätesten Zeit, einer Zeit, die erhalten wird durch Addieren eines Bereichs der Variation zu einer Zeit basierend auf dem ersten Zyklus.

## Revendications

1. Appareil de traitement d'information comportant :
un détecteur de rythme cardiaque (7) configuré pour mesurer le rythme cardiaque d'un utilisateur ;
un capteur d'accélération (9) configuré pour détecter une accélération ;
un dispositif de détection d'information de position (33) configuré pour détecter une information de position ;
un dispositif d'émission d'avertissement (29, 37) configuré pour émettre un avertissement ;
un dispositif de stockage (11) configuré pour conserver un premier cycle en fonction de temps de transition de profondeurs de sommeil pour un cycle ; et
un processeur (3) configuré pour détecter l'embarquement de l'utilisateur sur un moyen de transport en fonction de l'accélération, pour activer le capteur de rythme cardiaque lorsque l'embarquement a été détecté, pour détecter une transition vers le sommeil de l'utilisateur et un temps de transition vers le sommeil en fonction du rythme cardiaque mesuré par le capteur de rythme cardiaque, pour arrêter la commande du capteur de rythme cardiaque lorsque la transition a été détectée, et pour amener le dispositif d'émission d'avertissement à émettre un avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du premier cycle et d'un temps de transition vers le sommeil.

2. Appareil de traitement d'information selon la revendication 1, dans lequel :
le processeur (3) obtient en outre une instruction de démarrage de mesure pour calculer le premier cycle, active le capteur de rythme cardiaque après l'obtention de l'instruction de démarrage, effectue une mesure par le capteur de rythme cardiaque, et calcule le premier cycle en fonction du résultat de la mesure.

3. Appareil de traitement d'information selon la revendication 1 ou 2, dans lequel :
le processeur (3) commande le capteur de rythme cardiaque par intermittences conformément à un temps d'une transition vers une profondeur de sommeil prédite en fonction d'un temps de détection d'une transition vers la profondeur de sommeil et le premier cycle jusqu'à ce que les transitions vers les profondeurs de sommeil soient détectées pour un cycle après la détection d'une transition vers le sommeil après la détection de l'embarquement, calcule un second cycle en fonction des valeurs de mesure pour le premier cycle détecté, et émet l'avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du second cycle et d'un temps de transition vers le sommeil.

4. Appareil de traitement d'information selon la revendication 1 ou 2, dans lequel :
le processeur (3) commande le capteur de rythme cardiaque pendant une période de temps entre un premier temps et un dernier temps parmi les temps de transition prédits vers des profondeurs de sommeil calculées en fonction d'un temps auquel l'embarquement a été détecté, le premier cycle, et une variation prédite pour le premier cycle, modifie un temps de transition prédit de la profondeur de sommeil en fonction du temps de détection lorsqu'une transition vers la profondeur de sommeil a été détectée, et émet l'avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du temps de détection et d'un temps de transition vers le sommeil.

5. Appareil de traitement de l'information selon la revendication 4, dans lequel :
lorsqu'une transition vers la profondeur de sommeil n'est pas détectée avant un dernier temps parmi les temps de transition prédits, une période de temps d'un temps de transition prédit correspondant à une transition vers une profondeur de sommeil suivante se trouve entre un temps obtenu en ajoutant, au premier temps, un temps obtenu en réduisant une marge de la variation à partir d'un temps en fonction du premier cycle et un temps obtenu en ajoutant, au dernier temps, un temps obtenu en ajoutant une marge de la variation de temps en fonction du premier cycle.

6. Procédé d'émission d'avertissement, comportant :
la détection d'un embarquement d'un utilisateur sur un moyen de transport en fonction de l'accélération détectée par un capteur d'accélération (9) ;
l'activation d'un capteur de rythme cardiaque (7) ;
la détection d'une transition vers le sommeil de l'utilisateur en fonction du rythme cardiaque mesuré par le capteur de rythme cardiaque (7) ;
l'arrêt de la commande du capteur de rythme cardiaque (7) ;
la détermination si un processeur remplit ou non les conditions selon lesquelles une distance entre une position représentée par une information de position détectée par un dispositif de détection d'information de position (33) et une destination est plus courte qu'une valeur prescrite et qu'une profondeur de sommeil de l'utilisateur est peu profonde en fonction d'un premier cycle basé sur des temps de transmission des profondeurs de sommeil pour un cycle et un temps de transition vers le sommeil ; et
l'avertissement lorsque les conditions sont remplies.

7. Procédé d'émission d'avertissement selon la revendication 6, comportant en outre :
l'exécution d'une mesure par le capteur de rythme cardiaque en activant le capteur de rythme cardiaque et lorsqu'une instruction de démarrage de mesure pour calculer le premier cycle a été obtenue ; et
le calcul du premier cycle en fonction d'un résultat de la mesure par le processeur.

8. Procédé d'émission d'avertissement selon la revendication 6 ou 7, comportant en outre :
la détection d'une transition vers l'état de sommeil après la détection d'un embarquement ;
la commande du capteur de rythme cardiaque par intermittences conformément à un temps de transition vers une profondeur de sommeil prédite en fonction d'un temps de détection d'une transition vers la profondeur de sommeil et le premier cycle jusqu'à ce que les transitions vers les profondeurs de sommeil soient détectées pour un cycle ;
le calcul d'un second cycle en fonction des valeurs de mesure du premier cycle détectées ; et
l'émission de l'avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du second cycle et un temps de transition vers le sommeil par le processeur.

9. Procédé d'émission d'avertissement selon la revendication 6 ou 7, comportant en outre :
la commande du capteur de rythme cardiaque pendant une période de temps entre un premier temps et un dernier temps parmi les temps de transition prédits vers des profondeurs de sommeil calculées en fonction d'un temps auquel l'embarquement a été détecté, le premier cycle, et une variation prédite pour le premier cycle ;
la détection d'une transition vers la profondeur de sommeil ;
la modification d'un temps de transition prédit de la profondeur de sommeil en fonction du temps de détection ; et
l'émission de l'avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du temps de détection et d'un temps de transition vers le sommeil par le processeur.

10. Procédé d'émission d'avertissement selon la revendication 9, dans lequel :
lorsqu'une transition vers la profondeur de sommeil n'est pas détectée avant un dernier temps parmi les temps de transition prédits, une période de temps d'un temps de transition prédit correspondant à une transition vers une nouvelle profondeur de sommeil se trouve entre un temps obtenu en ajoutant, au premier temps, un temps obtenu en réduisant une marge de la variation à partir d'un temps en fonction du premier cycle et un temps obtenu en ajoutant, au dernier temps, un temps obtenu en ajoutant une marge à la variation à un temps en fonction du premier cycle.

11. Programme pour amener un ordinateur à exécuter un processus comportant :
la détection d'un embarquement d'un utilisateur sur un moyen de transport en fonction de l'accélération détectée par un capteur d'accélération (9) ;
l'activation d'un capteur de rythme cardiaque (7) ;
la détection d'une transition vers le sommeil de l'utilisateur en fonction du rythme cardiaque mesuré par le capteur de rythme cardiaque (7) ;
l'arrêt de la commande du capteur de rythme cardiaque (7) ;
la détermination si oui ou non les conditions selon lesquelles une distance entre une position représentée par une information de position détectée par un dispositif de détection d'information de position (33) et une destination est plus courte qu'une valeur prescrite et qu'une profondeur de sommeil de l'utilisateur est peu profonde en fonction d'un premier cycle basé sur des temps de transmission de profondeurs de sommeil pour un cycle et un temps de transmission vers le sommeil sont remplies ; et
l'émission d'un avertissement lorsque les conditions sont remplies.

12. Programme selon la revendication 11, le processus comportant en outre :
l'exécution d'une mesure par le capteur de rythme cardiaque en activant le capteur de rythme cardiaque lorsqu'une instruction de démarrage de mesure pour calculer le premier cycle a été obtenue ; et
le calcul du premier cycle en fonction d'un résultat de la mesure.

13. Programme selon la revendication 11 ou 12, le processus comportant en outre :
la détection d'une transition vers l'état de sommeil après la détection d'un embarquement ;
la commande du capteur de rythme cardiaque par intermittences conformément à un temps d'une transition vers une profondeur de sommeil prédite en fonction d'un temps de détection d'une transition vers la profondeur de sommeil et le premier cycle jusqu'à ce que les transitions vers les profondeurs de sommeil soient détectées pour un cycle ;
le calcul d'un second cycle en fonction de valeurs de mesure pour le premier cycle détecté ; et
l'émission de l'avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du second cycle et d'un temps de transition vers le sommeil.

14. Programme selon la revendication 11 ou 12, le processus comportant en outre :
la commande du capteur de rythme cardiaque pendant une période de temps entre un premier temps et un dernier temps parmi les temps de transition prédits vers des profondeurs de sommeil calculées en fonction d'un temps auquel l'embarquement a été détecté, le premier cycle, et une variation prédite pour le premier cycle ;
la détection d'une transition vers la profondeur de sommeil ;
la modification d'un temps de transition prédit de la profondeur de sommeil en fonction du temps de détection ; et
l'émission de l'avertissement lorsqu'il a été déterminé qu'une distance entre une position représentée par l'information de position détectée par le dispositif de détection d'information de position et une destination est plus courte qu'une valeur prescrite et qu'une profondeur de sommeil de l'utilisateur est peu profonde en fonction du temps de détection et d'un temps de transition vers le sommeil.

15. Programme selon la revendication 14, dans lequel:
lorsqu'une transition vers la profondeur de sommeil n'est pas détectée avant un dernier temps parmi les temps de transition prédits, une période de temps d'un temps de transition prédit correspondant à une transition vers une profondeur de sommeil suivante se trouve entre un temps obtenu en ajoutant, au premier temps, un temps obtenu en réduisant une marge de la variation à partir d'un temps en fonction du premier cycle et un temps obtenu en ajoutant, au dernier temps, un temps obtenu en ajoutant une marge à la variation à un temps en fonction du premier cycle.
